# FASCICULE DE BREVET EUROPEEN

(11) **EP 2 983 784 B1**
(45) Date de publication et mention de la délivrance du brevet: **11.09.2019**
(21) Numéro de dépôt: 14720662.7
(22) Date de dépôt: 04.04.2014
(51) Int. Cl.: A61K 38/17, A61P 3/10, A61P 3/04, A61P 5/50

(54) **COMPOSITION COMPRENANT LA PROTÉINE HIP/PAP OU L'UN DE SES DÉRIVÉS POUR LE TRAITEMENT DE LA RÉSISTANCE À L'INSULINE**
ZUSAMMENSETZUNG MIT HIP/PAP-PROTEIN ODER EINEM DERIVAT DAVON ZUR BEHANDLUNG VON INSULINRESISTENZ
COMPOSITION INCLUDING THE HIP/PAP PROTEIN OR ONE OF THE DERIVATIVES THEREOF FOR TREATING INSULIN RESISTANCE

(30) Priorité: 10.04.2013 FR 1353245
(43) Date de publication de la demande: 17.02.2016
(73) Titulaire: THE HEALTHY AGING COMPANY, 75001 Paris (FR)
(72) Inventeur: ANDREELLI, Fabrizio, F-92300 Levallois Perret (FR); AMOUYAL, Paul, F-92310 Sevres (FR); MAGNAN, Christophe, F-75020 Paris (FR); CRUCIANI-GUGLIELMACCI, Céline, F-78360 Montesson (FR); FAIVRE, Jamila, F-75016 Paris (FR); DARNAUD, Marion, 69003 Lyon (FR); JAMOT, Laure, 75013 Paris (FR); BRECHOT, Christian, F-75015 Paris (FR); AMOUYAL, Gilles, F-75016 Paris (FR)
(74) Mandataire: Nony
(86) Numéro de dépôt international: PCT/FR2014/050821
(87) Numéro de publication internationale: WO 2014/167230

(56) Documents cités:
- EP-A1- 2 260 857
- WO-A2-2006/128083
- OKAMOTO H: "THE REG GENE FAMILY AND REG PROTEINS: WITH SPECIAL ATTENTION TO THE REGENERATION OF PANCREATIC BETA-CELLS", JOURNAL OF HEPATO-BILIARY-PANCREATIC SURGERY, SPRINGER VERLAG, BERLIN, DE, vol. 6, no. 3, 1 janvier 1999 (1999-01-01) , pages 254-262, XP001203941, ISSN: 0944-1166, DOI: 10.1007/S005340050115
- ROSTY C ET AL: "IDENTIFICATION OF HEPATOCARCINOMA-INTESTINE-PANCREAS/PANCREA TITIS-ASSOCIATED PROTEIN I AS A BIOMARKER FOR PANCREATIC DUCTAL ADENOCARCINOMA BY PROTEIN BIOCHIP TECHNOLOGY", CANCER RESEARCH, AMERICAN ASSOCIATION FOR CANCER RESEARCH, US, vol. 62, no. 2, 15 mars 2002 (2002-03-15), pages 1868-1875, XP001179968, ISSN: 0008-5472
- OGAWA H ET AL: "Increased expression of HIP/PAP and regenerating gene III in human inflammatory bowel disease and a murine bacterial reconstitution model", INFLAMMATORY BOWEL DISEASES, WILLAMS AND WILKINS, HAGERSTOWN, MD, US, vol. 9, no. 3, 1 mai 2003 (2003-05-01), pages 162-170, XP002688917, ISSN: 1078-0998
- GRAF R ET AL: "Exocrine Meets Endocrine: Pancreatic Stone Protein and Regenerating Protein-Two Sides of the Same Coin", JOURNAL OF SURGICAL RESEARCH, ACADEMIC PRESS INC., SAN DIEGO, CA, US, vol. 133, no. 2, 15 juin 2006 (2006-06-15) , pages 113-120, XP024952707, ISSN: 0022-4804, DOI: 10.1016/J.JSS.2005.09.030 [extrait le 2006-06-15]
- ZHANG YU-WEI ET AL: "Reg gene family and human diseases", WORLD JOURNAL OF GASTROENTEROLOGY, WJG PRESS, CN, vol. 9, no. 12, 18 août 2011 (2011-08-18), pages 2635-2641, XP002657060, ISSN: 1007-9327

### SEQUENCE LISTING

<110> ALFACT INNOVATION
<120> Composition comprenant la protéine HIP/PAP ou l'un de ses dérivés
   pour le traitement de la résistance à l'insuline
<130> Y543 FR Alfact Innovation
<160> 4
<170> PatentIn version 3.5
<210> 1
   <211> 149
   <212> PRT
   <213> Homo sapiens
<400> 1
   Glu Glu Pro Gln Arg Glu Leu Pro Ser Ala Arg Ile Arg Cys Pro Lys
   1               5                   10                  15
   Gly Ser Lys Ala Tyr Gly Ser His Cys Tyr Ala Leu Phe Leu Ser Pro
               20                  25                  30
   Lys Ser Trp Thr Asp Ala Asp Leu Ala Cys Gln Lys Arg Pro Ser Gly
           35                  40                  45
   Asn Leu Val Ser Val Leu Ser Gly Ala Glu Gly Ser Phe Val Ser Ser
       50                  55                  60
   Leu Val Lys Ser Ile Gly Asn Ser Tyr Ser Tyr Val Trp Ile Gly Leu
   65                  70                  75                  80
   His Asp Pro Thr Gln Gly Thr Glu Pro Asn Gly Glu Gly Trp Glu Trp
                   85                  90                  95
   Ser Ser Ser Asp Val Met Asn Tyr Phe Ala Trp Glu Arg Asn Pro Ser
               100                 105                 110
   Thr Ile Ser Ser Pro Gly His Cys Ala Ser Leu Ser Arg Ser Thr Ala
           115                 120                 125
   Phe Leu Arg Trp Lys Asp Tyr Asn Cys Asn Val Arg Leu Pro Tyr Val
       130                 135                 140
   Cys Lys Phe Thr Asp
   145
<210> 2
   <211> 138
   <212> PRT
   <213> Homo sapiens
<400> 2
   Ile Arg Cys Pro Lys Gly Ser Lys Ala Tyr Gly Ser His Cys Tyr Ala
   1               5                   10                  15
   Leu Phe Leu Ser Pro Lys Ser Trp Thr Asp Ala Asp Leu Ala Cys Gln
               20                  25                  30
   Lys Arg Pro Ser Gly Asn Leu Val Ser Val Leu Ser Gly Ala Glu Gly
           35                  40                  45
   Ser Phe Val Ser Ser Leu Val Lys Ser Ile Gly Asn Ser Tyr Ser Tyr
       50                  55                  60
   Val Trp Ile Gly Leu His Asp Pro Thr Gln Gly Thr Glu Pro Asn Gly
   65                  70                  75                  80
   Glu Gly Trp Glu Trp Ser Ser Ser Asp Val Met Asn Tyr Phe Ala Trp
                   85                  90                  95
   Glu Arg Asn Pro Ser Thr Ile Ser Ser Pro Gly His Cys Ala Ser Leu
               100                 105                 110
   Ser Arg Ser Thr Ala Phe Leu Arg Trp Lys Asp Tyr Asn Cys Asn Val
           115                 120                 125
   Arg Leu Pro Tyr Val Cys Lys Phe Thr Asp
       130                 135
<210> 3
   <211> 150
   <212> PRT
   <213> Homo sapiens
<400> 3
   Met Glu Glu Pro Gln Arg Glu Leu Pro Ser Ala Arg Ile Arg Cys Pro
   1               5                   10                  15
   Lys Gly Ser Lys Ala Tyr Gly Ser His Cys Tyr Ala Leu Phe Leu Ser
               20                  25                  30
   Pro Lys Ser Trp Thr Asp Ala Asp Leu Ala Cys Gln Lys Arg Pro Ser
           35                  40                  45
   Gly Asn Leu Val Ser Val Leu Ser Gly Ala Glu Gly Ser Phe Val Ser
       50                  55                  60
   Ser Leu Val Lys Ser Ile Gly Asn Ser Tyr Ser Tyr Val Trp Ile Gly
   65                  70                  75                  80
   Leu His Asp Pro Thr Gln Gly Thr Glu Pro Asn Gly Glu Gly Trp Glu
                   85                  90                  95
   Trp Ser Ser Ser Asp Val Met Asn Tyr Phe Ala Trp Glu Arg Asn Pro
               100                 105                 110
   Ser Thr Ile Ser Ser Pro Gly His Cys Ala Ser Leu Ser Arg Ser Thr
           115                 120                 125
   Ala Phe Leu Arg Trp Lys Asp Tyr Asn Cys Asn Val Arg Leu Pro Tyr
       130                 135                 140
   Val Cys Lys Phe Thr Asp
   145                 150
<210> 4
   <211> 175
   <212> PRT
   <213> Homo sapiens
<400> 4
   Met Leu Pro Pro Met Ala Leu Pro Ser Val Ser Trp Met Leu Leu Ser
   1               5                   10                  15
   Cys Leu Met Leu Leu Ser Gln Val Gln Gly Glu Glu Pro Gln Arg Glu
               20                  25                  30
   Leu Pro Ser Ala Arg Ile Arg Cys Pro Lys Gly Ser Lys Ala Tyr Gly
           35                  40                  45
   Ser His Cys Tyr Ala Leu Phe Leu Ser Pro Lys Ser Trp Thr Asp Ala
       50                  55                  60
   Asp Leu Ala Cys Gln Lys Arg Pro Ser Gly Asn Leu Val Ser Val Leu
   65                  70                  75                  80
   Ser Gly Ala Glu Gly Ser Phe Val Ser Ser Leu Val Lys Ser Ile Gly
                   85                  90                  95
   Asn Ser Tyr Ser Tyr Val Trp Ile Gly Leu His Asp Pro Thr Gln Gly
               100                 105                 110
   Thr Glu Pro Asn Gly Glu Gly Trp Glu Trp Ser Ser Ser Asp Val Met
           115                 120                 125
   Asn Tyr Phe Ala Trp Glu Arg Asn Pro Ser Thr Ile Ser Ser Pro Gly
       130                 135                 140
   His Cys Ala Ser Leu Ser Arg Ser Thr Ala Phe Leu Arg Trp Lys Asp
   145                 150                 155                 160
   Tyr Asn Cys Asn Val Arg Leu Pro Tyr Val Cys Lys Phe Thr Asp
                   165                 170                 175

## Description

### ETAT DE LA TECHNIQUE

La présente invention se rapporte à l'utilisation de la protéine HIP/PAP ou de l'un de ses dérivés, pour réduire la résistance à l'insuline ou pour prévenir son développement. L'invention sera particulièrement destinée aux patients non insulino-requérants.

La résistance à l'insuline est définie par la diminution des effets métaboliques de l'insuline sur les tissus insulino-sensibles (muscles squelettiques, tissu adipeux blanc, foie). En pratique, l'insulino-résistance (IR) se manifeste par une incapacité de l'insuline à inhiber la production de glucose hépatique (via l'inhibition de la néoglucogénèse ou de la glycogénolyse), par une diminution de la capacité du tissu musculaire à capter, et donc à utiliser le glucose, et par une lipolyse exagérée du tissu adipeux blanc entraînant une élévation de la concentration plasmatique d'acides gras libres.
Avec la sécrétion d'insuline, la sensibilité à l'insuline (ou à l'inverse la résistance à l'insuline), sont deux variables dépendantes, qui interagissent de concert pour maintenir l'homéostasie glycémique. Toute modification de la glycémie entraîne une modification de l'insulinémie et inversement. En conséquence, toute augmentation de l'insulino-résistance est compensée par l'augmentation concomitante de la sécrétion d'insuline (on observe alors une hyperinsulinémie compensatoire) de façon à maintenir la glycémie normale (normoglycémie) ou limiter l'évolution vers une hyperglycémie (i.e. éviter l'escalade pathologique de la tolérance glycémique normale, vers la glycémie modérée à jeun ou l'intolérance au glucose). A partir d'un certain seuil, la progression de l'IR entraîne un phénomène d'échappement dû à l'épuisement de la fonction insulino-sécrétoire bêta pancréatique, dont les mécanismes sous-jacents sont encore complexes (perte fonctionnelle et structurelle de la fonction mitochondriale et/ou apoptose des cellules bêta pancréatiques notamment liée à une glucotoxicité). La glycémie augmente alors franchement (glycémie à jeun ≥ 1,26 g/L soit 7 mM), et l'on parle de diabète. On considère que le diabète de type II est installé, lorsque la glycémie dépasse ce seuil de 7 mM à jeun à deux reprises sur un prélèvement veineux. Ceci traduit la non-compensation de l'IR par la sécrétion d'insuline témoignant de l'existence d'une insulinopénie dite relative. A ce stade, il peut être nécessaire d'initier une insulinothérapie en complément de la prise en charge de l'IR, par les mesures hygiéno-diététiques, ou des traitements pharmacologiques. A un stade avancé, l'insulino-sécrétion dépendante du glucose devenant trop faible (voire quasi nulle), il devient alors impératif de mettre en place une insulinothérapie par multi-injections (see Silvio E et al., Diabetes Care 35:1364-1379, 2012; Management of Hyperglycemia in Type 2 Diabetes: A Patient-Centered Approach. Position Statement of the American Diabetes Association (ADA) and the European Association for the Study of Diabetes (EASD)).

L'insuline est dosée chez l'homme par méthode radio-immunologique, ou ELISA, à jeun sur du plasma ou du sérum. Les valeurs basales (à jeun) normales vont de 3,2 à 16,3 mUI/L, puis augmentent jusqu'à 40 mUI/L, dès la 20ème minute après une charge orale en glucose de 75 g, et retournent à des valeurs basales deux heures après l'ingestion de glucose. Cette mesure combinée à celle de la glycémie permet d'estimer la capacité de l'insuline à promouvoir la captation tissulaire du glucose et maintenir ainsi une homéostasie glucidique normale.
La sensibilité à l'insuline (ainsi que la fonction sécrétoire pancréatique β qui lui est corrélée) peut être estimée en utilisant le logiciel HOMA calculator (v. 2.2.2, Diabetes trials Unit, University of Oxford, téléchargeable à l'adresse suivante : http://www.dtu.ox.ac.uk/homacalculator/download.php) récemment affiné en HOMA2 (*Homeostatic Model Assesment*). Le modèle HOMA 2 (Levy JC et al., Diabetes Care, 1998; 21: 2191-2192, voir également Wallace TM, Levy JC, Matthews DR. Diabetes Care, 2004; 27(6): 1487-95) est un modèle structural informatique, dont le calculateur est basé sur la boucle de régulation glucose/insuline, et permet de déterminer à partir de mesures des concentrations plasmatiques à jeun de glucose et d'insuline (ou de peptide C), la fonction pancréatique béta (%β, i.e. HOMA B) et la sensibilité à l'insuline (% S) d'un individu, puisque pour chaque combinaison de % β et de %S, il n'existe qu'une seule combinaison de glucose et d'insuline/peptide C correspondante. La validation de ce calculateur a été effectuée en comparant les équations utilisées aux valeurs obtenues par la mesure directe de la sensibilité à l'insuline et de la sécrétion d'insuline par des méthodes invasives et réservées à la recherche (méthodes de clamp euglycémique hyperinsulinémique ou de clamp hyperglycémique). Le calculateur permet d'estimer à la fois la sensibilité à l'insuline et la sécrétion d'insuline par des prélèvements sanguins à jeun.
Il a été montré (Kahn SE et al., Diabetes 1993), que chez un sujet avec une tolérance glucidique normale, et des degrés variables d'obésité ou d'insulinorésistance, la fonction sécrétoire β variait de manière quantitative avec la sensibilité à l'insuline. Il existe en effet une relation hyperbolique entre la sensibilité à l'insuline (S) et la fonction β (β). Le produit de la valeur de l'ordonnée (β) par la valeur de l'abscisse S est une constante qui correspond à la tolérance glucidique du sujet.
Ainsi, il est possible d'estimer la résistance à l'insuline par le calcul de l'indice HOMA- IR (Matthews et al., 1985), selon la formule suivante : [(Insulinémie_{à jeun (µU/ml)} x Glycémie_{à jeun (mM)}) / 22,5]. Chez un sujet sain, non insulino-résistant, l'indice HOMA-IR à jeun est généralement proche de 1.
Il est enfin possible de réaliser un test de tolérance à l'insuline (ITT), qui consiste à suivre la glycémie après avoir injecté (par voie péritonéale, intraveineuse ou sous-cutanée) une dose d'insuline d'action rapide calculée sur le poids corporel (généralement 0,75 UI/kg de poids corporel). Cette technique est surtout pratiquée chez l'animal. Chez l'homme, afin de s'affranchir des difficultés et des risques liés à l'hypoglycémie, plusieurs aménagements ont été proposés, définissant un test de tolérance à l'insuline modifié ou test court par voie intraveineuse. L'interprétation ne porte que sur la vitesse de décroissance des glycémies sur les quinze premières minutes du test, avec détermination du rapport [(Glycémie basale _{G0} - Glycémie à 15 minutes_{G15}) / G0]. Ce rapport est normalement inférieur à 0,5 chez des patients sains, non insulino-résistants.

La concentration sérique de peptide C est un marqueur de la sécrétion d'insuline et reflète la capacité des cellules béta pancréatiques à produire l'insuline. Les valeurs normales à jeun vont de 0,27 à 1,43 nmol/L (ou de 0,8 à 4,2 ng/mL) chez l'homme. Chez un sujet présentant un diabète de type I ou un diabète de type II avec atteinte de cellules pancréatiques, le taux de peptide C est diminué. Chez les patients présentant une résistance à l'insuline associée à une hyperinsulinémie endogène, ce taux est augmenté par rapport à la normale.

Les étiologies possibles de la résistance à l'insuline sont nombreuses et résultent de plusieurs anomalies éventuellement combinées : un surpoids abdominal, ou une obésité viscérale, une sédentarité prolongée ou une inactivité physique, des anomalies génétiques ou acquises affectant le fonctionnement des protéines de la cascade de signalisation post-récepteurs de l'insuline, un excès d'hormones contre-régulatrices, un dysfonctionnement ovarien, divers agents pharmaceutiques (comme par exemple les corticothérapies, l'abus de stéroïdes sexuels), la gestation, les situations d'hypercatabolisme, la dénutrition, un poids de naissance faible pour la taille et/ou une malnutrition foetale. Il a été montré en particulier que l'obésité était un facteur majeur de l'IR et que cette dernière augmentait lorsque l'indice de masse corporelle (IMC) augmentait. En particulier, l'IR est quasiment certaine, lorsque l'IMC dépasse 40 kg/m² (Mericq et al.). En complément, il a été montré que la distribution de l'excès de masse grasse joue également un rôle dans la mise en place de l'IR. En effet, l'obésité abdominale (ou tronculaire ou viscérale) est reconnue comme fondamentale dans la physiopathologie de l'IR à l'échelon de l'organisme entier. Ceci explique la possibilité d'être insulino-résistant pour des IMC largement inférieurs à 40 kg/m2 voire dans les valeurs du surpoids chez des sujets, si l'excès de masse grasse est abdominal. Entrent dans cette catégorie, les patients ayant eu un évènement cardiovasculaire (infarctus du myocarde, accident vasculaire cérébral ischémique, artériopathie des membres inférieurs), qui fait découvrir l'état d'insulinorésistance jusqu'alors méconnu. En effet, ces populations atteintes d'insulinorésistance sont à haut risque de survenue d'un évènement cardiovasculaire indépendamment des facteurs de risque classique (LDL-cholestérol élevé, tabagisme, hypertension artérielle, diabète de type 2). La survenue d'un évènement cardiovasculaire dans cette population doit faire diagnostiquer l'insulinorésistance afin de la prendre en charge sur le plan thérapeutique. La plupart des situations où l'insulinorésistance est présente ont en commun une altération de la distribution du stockage de la masse grasse. Le tissu adipeux joue en effet un rôle important dans le stockage de l'excès énergétique provenant des aliments sous forme d'acide gras. Sa masse accrue dans l'obésité, réduit la captation du glucose et augmente la lipolyse. Chez les sujets prédisposés à l'IR, on observe un développement du tissu adipeux intra-abdominal. Celui-ci est lui-même moins sensible que le tissu adipeux sous-cutané pour l'effet d'inhibition de la lipolyse par l'insuline. En conséquence, le tissu adipeux viscéral a des capacités lipolytiques accrues. Les acides gras libérés en excès et de manière mal régulée sont récupérés par la circulation péritonéale puis la veine porte puis par le foie. Cet afflux d'acides gras participe en grande partie à la physiopathologie de la stéatose hépatique non alcoolique des sujets insulino-résistants. Le foie stéatosique exporte en partie les lipides stockés sous forme de triglycérides (particules VLDL-triglycérides) dans le sang circulant. Ces triglycérides peuvent alors être hydrolysés par la lipoprotéine lipase (LPL) endothéliale ce qui permet aux muscles squelettiques et aux îlots pancréatiques de capter ces acides gras. Il apparaît ainsi des dépôts multi-tissulaires de lipides ectopiques, qui eux-mêmes participent à la réduction de l'effet de l'insuline et/ou à la réduction de la sécrétion d'insuline.

L'IR est donc associée à divers états pathologiques tels que les dyslipidémies et/ou l'hypertension artérielle. Le phénotype associé à l'IR est ainsi considéré comme un acteur central dans le développement des maladies cardiovasculaires athéromateuses.

La résistance à l'insuline représente désormais une cible thérapeutique à part entière. Ainsi, dans le cadre du risque de survenue d'un évènement cardiovasculaire s'il est recommandé de contrôler les facteurs cardiovasculaires classiques (hypertension artérielle, dyslipidémie athérogène, tabagisme, diabète de type 2), il est également de plus en plus indiqué d'intervenir directement sur la résistance à l'insuline.
En outre, on considère actuellement que l'hyperinsulinisme chronique provoqué par l'IR peut favoriser la mitogenèse cellulaire expliquant un sur-risque de survenue de cancers dans les populations insulino-résistantes.

Il apparaît donc fondamental de développer des outils thérapeutiques permettant de cibler spécifiquement la résistance à l'insuline, et non l'insulinopénie et/ou les déficits fonctionnels bêta pancréatiques, pour traiter des patients, en particulier en amont du diabète de type II, et plus particulièrement des patients non insulino-requérants.

Les antidiabétiques oraux connus, comme les sulfamides hypoglycémiants ou sulfonylurés et les glinides agissent exclusivement sur les cellules bêta du pancréas endocrine en stimulant l'insulino-sécrétion, mais ils n'ont aucun effet direct sur la sensibilité à l'insuline. On connaît actuellement des composés permettant de réduire spécifiquement l'insulino-résistance (sans effet sur l'insulino-sécrétion), comme les thiazolidinediones, ou les glitazones, ou encore, dans une moindre mesure, la metformine, qui agissent respectivement sur l'insulino-résistance adipeuse et hépatique. Néanmoins leur utilisation est délicate en raison des effets secondaires importants ou de la difficulté de les employer chez certains patients (intolérance digestive, grossesse, insuffisance rénale ou cardiaque ou hépatique, etc.).

Il existe donc un besoin de nouveaux composés pour traiter, diminuer ou prévenir la résistance à l'insuline chez des patients non insulino-requérants, ou non insulino-péniques. Et ce d'autant que la classe des glitazones n'est plus commercialisée en France réduisant la classe des hypoglycémiants insulino-sensibilisateurs à la seule metformine.

La demanderesse a démontré, que de façon surprenante, la protéine HIP/PAP permettait de diminuer spécifiquement la résistance à l'insuline. La protéine HIP/PAP peut donc avantageusement être utilisée pour traiter, limiter ou prévenir l'IR et les symptômes et troubles associés, en particulier chez des patients non insulino-requérants.
La protéine HIP/PAP est connue pour son activité anti-apoptotique et mitogénique sur les cellules hépatiques (US 13/032,521, WO 2004/112824, Simon et al., FASEB J. 2003 Aug;17(11):1441-50).
Il a également été montré qu'un peptide de 15 acides aminés, dérivé de la famille Reg IIIa (HIP/PAP), le peptide HIP (Human prolslet Peptide) possédait une activité régénératrice sur les îlots pancréatiques et stimulait ainsi la production d'insuline (US 2010/0093605). Sur la base de ces résultats il a été suggéré l'utilisation dudit peptide et de dérivés de protéines de la famille Reg IIIa pour le traitement de patients strictement insulino-requérants (atteints de diabète de type I ou présentant un diabète de type II décompensé).

A la connaissance de la demanderesse, aucun document de l'art antérieur ne décrit l'effet de la protéine HIP/PAP sur la résistance à l'insuline, pour son utilisation chez des sujets non insulino-requérants.

### RESUME DE L'INVENTION

La présent texte décrit la protéine HIP/PAP ou l'un de ses dérivés pour son utilisation, chez des sujets non insulino-requérants, pour le traitement ou la prévention de la résistance à l'insuline.

La présente invention se rapporte donc à la protéine HIP/PAP ou l'un de ses dérivés pour son utilisation, chez des sujets non insulino-requérants, pour le traitement ou la prévention de la résistance à l'insuline caractérisée en ce que ledit dérivé comprend une séquence en acides aminés ayant une identité de séquence d'au moins 90% avec le polypeptide constitué de la séquence d'acides aminés choisie parmi les séquences SEQ ID No 1 à 4.Le présent texte décrit également la protéine HIP/PAP ou l'un de ses dérivés pour limiter, et/ou réduire la résistance à l'insuline ou pour prévenir son apparition dans les populations à risque. Est également décrite la protéine HIP/PAP, ou l'un de ses dérivés, pour son utilisation chez des sujets non insulino-requérants, pour le traitement ou la prévention de la résistance à l'insuline des tissus périphériques.

Selon différents modes de réalisation, les sujets cibles de l'invention présentent une fonction pancréatique β (%β, i.e. : HOMA B), estimée à partir du modèle HOMA 2, supérieur ou égale à 60 % et/ou un HOMA-IR inférieur à 6.
En particulier, la protéine HIP/PAP, ou l'un de ses dérivés, peut être destinée à des sujets présentant une glycémie à jeun inférieure à 125 mg/dL, notamment inférieure à 120 mg/dL et plus particulièrement inférieure à 110 mg/dL.
Dans certains modes de réalisation, les sujets présentent, 2h après un test de tolérance oral au glucose, une glycémie inférieure à 200 mg/dL, notamment inférieure à 180 mg/dL et plus particulièrement inférieure à 140 mg/dL.
Dans certains modes de réalisation, les sujets ont une tolérance glucidique normale et/ou sont normo-glycémiques à jeun.

La présente invention trouvera également des applications pour augmenter le développement musculaire, stimuler la prise de masse maigre et prévenir ou limiter le catabolisme musculaire et la dénutrition protéique, pour prévenir ou traiter la dyslipidémie et en particulier l'hypercholestérolémie, pour prévenir ou traiter l'athérosclérose, en particulier la maladie coronarienne ou cérébrale, et l'artériopathie des membres inférieurs.

Ainsi, la présente invention a également pour objet la protéine HIP/HAP, ou l'un de ses dérivés, pour son utilisation chez des sujets non insulino-requérants, pour augmenter le développement musculaire, stimuler la prise de masse maigre et prévenir ou limiter le catabolisme musculaire et la dénutrition protéique, caractérisée en ce que ledit dérivé comprend une séquence en acides aminés ayant une identité de séquence d'au moins 90% avec le polypeptide constitué de la séquence d'acides aminés choisie parmi les séquences SEQ ID No 1 à 4.

La présente invention a en outre pour objet la protéine HIP/HAP, ou l'un de ses dérivés, pour son utilisation chez des sujets non insulino-requérants, pour prévenir ou traiter la dyslipidémie et en particulier l'hypercholestérolémie, caractérisée en ce que ledit dérivé comprend une séquence en acides aminés ayant une identité de séquence d'au moins 90% avec le polypeptide constitué de la séquence d'acides aminés choisie parmi les séquences SEQ ID No 1 à 4..

La présente invention concerne également la protéine HIP/HAP, ou l'un de ses dérivés, pour son utilisation chez des sujets non insulino-requérants, pour prévenir ou traiter l'athérosclérose et en particulier la maladie coronarienne et l'artériopathie des membres inférieurs, caractérisée en ce que ledit dérivé comprend une séquence en acides aminés ayant une identité de séquence d'au moins 90% avec le polypeptide constitué de la séquence d'acides aminés choisie parmi les séquences SEQ ID No 1 à 4.

Ainsi, le présent texte sera également avantageusement destiné aux sujets présentant au moins l'un des troubles choisi parmi un surpoids viscéral, une obésité, un syndrome métabolique, un syndrome des ovaires polykystiques, un trouble de l'alimentation, une hépatite C, une stéatose hépatique, une sarcopénie, et plus particulièrement aux sujets présentant au moins un trouble choisi parmi le groupe constitué par le surpoids viscéral, l'obésité, l'hyperandrogénie, un trouble de l'alimentation, la sarcopénie, l'hypercatabolisme, la dénutrition. La présente invention est par ailleurs avantageusement destinée aux sujets présentant au moins l'un des troubles choisi parmi le groupe constitué par : le surpoids viscéral, l'obésité, l'hyperandrogénie, un trouble de l'alimentation, la sarcopénie, l'hypercatabolisme, la dénutrition.

La protéine HIP/PAP, ou l'un de ses dérivés, selon l'invention est également destinée au traitement de l'insulino-résistance associée au vieillissement. Dans certains modes de réalisation de l'invention, le sujet non insulino-requérant est donc un sujet âgé.

Les dérivés de la protéine HIP/PAP sont caractérisés en ce qu'ils comprennent une séquence en acides aminés ayant une identité de séquence d'au moins 90 % avec le polypeptide constitué de la séquence d'acides aminés choisie parmi les séquences SEQ ID N°1 à 4. La séquence desdits dérivés diffère de la séquence SEQ ID N°4 par au moins 1 acide aminé.

L'invention se rapporte enfin à une composition comprenant une quantité efficace de la protéine HIP/PAP ou de l'un de ses dérivés, telle que définie selon la présente invention, en mélange avec au moins un excipient physiologiquement acceptable, pour son utilisation pour traiter ou prévenir l'insulino-résistance chez le sujet non insulino-requérant.
Les compositions selon l'invention pourront comprendre une quantité efficace de la protéine HIP/PAP ou de l'un de ses dérivés, en association avec un autre agent actif pour réguler l'homéostasie glycémique.
Les différents modes de réalisation de l'invention pourront être pris, ou non, en combinaison les uns avec les autres.

### DESCRIPTION DETAILLEE DE L'INVENTION

La demanderesse a mis en évidence que l'administration de la protéine HIP/PAP à des sujets non insulino-requérants, mais présentant des troubles de l'homéostasie glucidique, entraîne une diminution très significative de l'indice HOMA-IR ainsi qu'une augmentation de l'effet hypoglycémiant de l'insuline exogène, ce qui se traduit par une plus grande chute de la glycémie, après administration intra-péritonéale d'insuline d'action rapide lors d'un test de tolérance à l'insuline (ou ITT). Cet effet suggère que l'administration de la protéine HIP/PAP entraine une réduction spécifique de l'insulinorésistance. Ces effets ne s'accompagnent pas d'une augmentation de la sécrétion d'insuline, ni au niveau basal, ni lors de tests OGTT. Ces résultats illustrent ainsi un effet spécifique de la protéine HIP/PAP sur la diminution de la résistance à l'insuline (ou sur l'augmentation de la sensibilité à l'insuline) et non sur l'insulino-sécrétion.

### Catégories de sujets et application thérapeutique de l'invention:

Le présent texte décrit la protéine HIP/PAP, ou l'un de ses dérivés, pour son utilisation pour traiter, ou réduire la résistance à l'insuline, et la résistance des tissus périphériques, ou prévenir, limiter ou empêcher leur développement (et inversement pour augmenter la sensibilité à l'insuline, ou limiter ou prévenir la diminution de la sensibilité à l'insuline), chez des sujets non insulino-requérants.
Avantageusement la protéine HIP/PAP sera également utilisée pour stimuler ou augmenter l'assimilation tissulaire du glucose par les tissus cibles.

Selon l'invention, les sujets consistent en des mammifères, en particulier des êtres humains, des animaux domestiques et d'élevage ainsi que les animaux de zoo et de sport, comme les chiens, les chats, les chevaux, les veaux, les vaches, les boeufs, les cochons, les lapins, etc. Plus particulièrement, la présente invention est destinée aux êtres humains. Les valeurs données dans le présent texte, en définition des différents paramètres de la demande, sont fournies en référence aux valeurs moyennes retrouvées chez l'homme en bonne santé.

Par sujets non insulino-requérants, on entend un sujet pour lequel le maintien de l'homéostasie glycémique ne nécessite pas l'administration d'insuline. Plus particulièrement, les patients non insulino-requérants ne suivent aucun traitement avec des antidiabétiques oraux de la famille des insulino-sécréteurs. A titre d'exemple, un insulino-sécréteur comprend notamment les sulfamides hypoglycémiants ou sulfonylurés et les glinides. Chez de tels patients en effet, la fonction sécrétoire bêta pancréatique est maintenue (absence d'insulinopénie endogène), de sorte qu'une insulinothérapie exogène n'est pas administrée.

Le présent texte décrit plusieurs modes de réalisation définis ci-dessous, qui pourront être pris en combinaison les uns avec les autres.
En particulier, l'invention s'adresse à des sujets ayant une fonction pancréatique β (%β ; c'est-à-dire HOMA B), estimée à partir du modèle HOMA 2 supérieure ou égale à 60 %, en particulier supérieure ou égale à 70 %, supérieure ou égale à 75 %, ou supérieure ou égale à 80 %. Cette valeur ne peut être supérieure à 100 %. De tels sujets présentent également un HOMA-IR inférieur à 6 et plus particulièrement inférieur ou égal à 5, notamment inférieur ou égal à 4,5. Selon l'invention, l'indice HOMA-IR est également supérieur ou égal à 0,8 et notamment supérieur ou égal à 0,9. Par exemple la protéine HIP/PAP peut être administrée à des sujets présentant un indice HOMA-IR variant de 0,8 à 5, ou encore de 0,9 à 4,5, plus particulièrement de 0,9 à 3.

L'invention pourra ainsi être appliquée à des sujets présentant une glycémie variable inférieure à 126 mg/dL à jeun. En particulier, l'invention n'est pas destinée aux diabétiques.
Les sujets de l'invention pourront donc présenter une glycémie normale (sujets normoglycémiques à jeun), c'est-à-dire inférieure à 110 mg/dL à jeun, de préférence inférieure ou égale à 100 mg/dL, notamment variant entre 80 à 100 mg/dL, ou encore inférieure ou égale à 95 mg/dL et notamment variant de 80 à 95 mg/dL à jeun. Les sujets de l'invention pourront selon un autre aspect présenter une hyperglycémie modérée à jeun inférieure à 125 mg/dL à jeun, notamment inférieure ou égale à 120 mg/dL, en particulier variant de 110 à 125 mg/dL, plus particulièrement de 110 à 120 mg/dL à jeun.

Dans certains modes de réalisation, les sujets de l'invention, présentant une hyperglycémie modérée à jeun, peuvent également être intolérants au glucose et présenter ainsi à la 2^{ème} heure d'une charge orale de 75 g de glucose, une glycémie inférieure à 200 mg/dL et notamment comprise entre 140 et 200 mg/dL. Pour des valeurs inférieures à 140 mg/dL à la 2^{ème} heure de la charge orale de 75 g de glucose, on considère généralement que la tolérance glucidique est normale.

La protéine HIP/PAP pour son utilisation selon l'invention sera également particulièrement destinée à des sujets tels que définis, à risques de développer une insulino-résistance, c'est-à-dire présentant par exemple l'une au moins des conditions pathologiques suivantes, favorables au développement d'une insulino-résistance : un surpoids, notamment viscéral ou une obésité (notamment viscérale), une anomalie de distribution de la masse grasse telle que les lipoatrophies partielles ou généralisées, acquises ou congénitales, les lipohypertrophies partielles ou totales, acquises ou congénitales, les anomalies de distribution de la masse grasse faisant parties d'un syndrome génétique d'IR (tels que le syndrome de Dunningan, le syndrome de Köbberling, le syndrome de Barraquer et Simons, le syndrome de Launsois-Bensaude ou lipomatose symétrique proximale, le syndrome de Rabson-Mendenhall), une hyperandrogénie, un trouble de l'alimentation, une diminution de la masse et de la force musculaire (autrement dit une sarcopénie), une sédentarité prolongée ou une inactivité physique, des anomalies génétiques ou acquises affectant le fonctionnement des protéines de la cascade de signalisation post-récepteurs de l'insuline, un excès d'hormones contre-régulatrices, un dysfonctionnement ovarien, divers agents pharmaceutiques (comme par exemple les corticothérapies ou les perturbations de la sécrétion endogène du cortisol, l'abus de stéroïdes sexuels, les antirétroviraux), un évènement cardiovasculaire révélateur de l'insulino-résistance, une situation d'hypercatabolisme, un poids de naissance faible pour la taille et/ou une malnutrition foetale.
Le surpoids viscéral ou abdominal fait référence à une répartition de type androïde, dans laquelle l'excès de graisse se situe au niveau des organes abdominaux, sur la paroi abdominale et parfois sur le haut du dos. Le surpoids (ou surcharge pondérale) et l'obésité sont couramment définis par un Indice de Masse Corporelle (IMC), qui prend en compte le poids et la taille d'un sujet pour catégoriser sa corpulence. Exprimé en kg/m², l'IMC correspond au poids divisé par le carré de la taille. Selon les normes de l'OMS, pour un être humain adulte, un IMC compris entre 18,5 et 25 kg/m² correspond à une corpulence dite « normale», c'est-à-dire non associée à une augmentation de la charge de morbidité (effets indésirables sur la santé). Le surpoids est caractérisé par un IMC compris entre 25 et 30 kg/m², et l'obésité par un IMC supérieur à 30 kg/m².

Selon un mode de réalisation préféré de l'invention, la protéine HIP/PAP sera utilisée pour prévenir, en particulier pour réduire ou limiter le développement de l'insulino-résistance, chez des sujets non insulino-péniques et/ou intolérants au glucose et/ou normoglycémiques. De tels sujets pourront présenter au moins un des facteurs de risque tels que rapportés ci-dessus.

La demanderesse a également démontré que la protéine HIP/PAP présentait un effet significatif sur les dyslipidémies, et permettait notamment de réduire les concentrations sériques de cholestérol et/ou de triglycérides chez les sujets normaux, ou chez des sujets dont le poids est supérieur à la normale (surpoids ou obésité). La protéine HIP/PAP sera donc avantageusement utilisée chez des sujets présentant une dyslipidémie et notamment une hypercholestérolémie (taux sérique de cholestérol supérieur ou égal à 2 g/L) et/ou une hypertriglycéridémie (taux sérique de triglycérides supérieur ou égal à 2,3 mmol/L). La protéine HIP/PAP sera également utilisée selon l'invention chez des patients ayant subi un accident cardiovasculaire ou présentant une athérosclérose, une maladie coronarienne et/ou une artériopathie des membres inférieurs.
Selon certains modes de réalisation, la protéine HIP/PAP selon l'invention sera ainsi utilisée pour traiter, réduire l'hyperlipémie, et en particulier l'hypercholestérolémie ou pour prévenir ou limiter leur développement. Plus particulièrement, l'utilisation de la protéine HIP/PAP ou de l'un de ses dérivés selon l'invention permettra de réduire les taux sériques de LDL-cholestérol ou de limiter, réduire ou empêcher leur augmentation. La protéine HIP/PAP de l'invention sera également adaptée au traitement et à la prévention des pathologies associées telles l'athérosclérose, la maladie coronarienne et l'artériopathie des membres inférieurs.

Enfin la demanderesse a mis en évidence que la protéine HIP/PAP entraine, chez les sujets normaux, comme chez les sujets en surpoids et/ou suivant un régime hyperlipidique, une diminution du ratio masse grasse / masse maigre. Plus particulièrement, le traitement avec la protéine HIP/PAP entraine, chez les sujets en surpoids et/ou suivant un régime hyperlipidique, une diminution significative de la masse grasse, associée à une tendance à l'augmentation de la masse maigre. Chez les sujets normaux, l'administration de HIP/PAP entraine une augmentation significative de la masse maigre associée à une tendance à la diminution de la masse grasse. Cet effet n'est pas associé à une perte de poids. Ces résultats démontrent que HIP/PAP influence le métabolisme énergétique et modifie la composition corporelle en diminuant le ratio masse grasse / masse maigre chez les sujets normaux comme chez les sujets en surpoids. Il a enfin été montré que la protéine HIP/PAP augmente l'assimilation du glucose spécifiquement par les muscles squelettiques en particulier chez les sujets ayant un régime hyper-lipidique et/ou en surpoids.

Les compositions de l'invention sont également particulièrement utiles pour traiter ou prévenir la résistance à l'insuline des tissus périphériques. On entend notamment par tissus périphériques, le foie, le tissu adipeux et les tissus musculaires squelettiques. L'augmentation de la sensibilité à l'insuline de ces tissus se traduit, entres autres, par une amélioration de leur capacité à assimiler le glucose.

L'augmentation de l'assimilation du glucose au niveau musculaire favorise le développement musculaire. En outre, il est connu qu'une diminution du rapport masse grasse / masse maigre influence favorablement l'évolution de pathologies du métabolisme énergétique, comme le syndrome métabolique, l'intolérance au glucose, le syndrome des ovaires polykistiques, l'hyperandrogénie ou les diabètes.
Le présent texte décrit donc également l'utilisation de la protéine HIP/PAP pour diminuer le rapport masse grasse / masse maigre ; elle s'applique en particulier chez des sujets âgés et/ou atteints de troubles du métabolisme énergétique et/ou chez des sujets dénutris et/ou présentant une sarcopénie et/ou un hypercatabolisme et/ou ayant un antécédent chirurgical significatif (comme une chirurgie bariatrique et/ou une dérivation intestinale et/ou une réduction de la sécrétion exocrine du pancréas post-chirurgicale et/ou une résection intestinale et/ou la nécessité post-chirurgicale d'une alimentation par support nutritionnel artificiel). Par sujet âgé, on entend notamment les sujets de plus de 60 ans en particulier, les sujets de plus de 70 ans et plus particulièrement les sujets de 75 ans et plus. Chez ces sujets, la protéine HIP/PAP sera également utile pour augmenter l'anabolisme et le développement musculaire, prévenir ou traiter la dénutrition en particulier protéique, stimuler la prise de masse maigre et prévenir et/ou limiter le catabolisme musculaire ou la sarcopénie, en particulier du sujet âgé. Avantageusement la protéine HIP/PAP ou l'un de ses dérivés selon le présent texte sera utilisée pour traiter, réduire ou limiter la fonte musculaire ou la sarcopénie associée au vieillissement chez le sujet âgé, ou à la dénutrition protéique.
La protéine HIP/PAP sera également avantageusement utilisée pour le traitement ou la prévention de la dénutrition ou de l'hyper-catabolisme. Par exemple, la protéine HIP/PAP pourra être administrée chez des sujets dénutris ou chez des patients en état hyper-catabolique, ou à risque de l'être, indépendamment de l'IMC des sujets, c'est-à-dire chez les sujets ayant un poids normal ou présentant un surpoids ou une obésité. En effet, la dénutrition touche également les sujets en surpoids ou obèses.

Le présent texte décrit donc également un intérêt pour l'utilisation de l'invention pour le traitement de l'insulino-résistance associée au vieillissement. La protéine HIP/PAP selon l'invention sera ainsi avantageusement utilisée, en particulier chez des sujets âgés, et/ou pour augmenter le développement musculaire, et/ou stimuler la prise de masse maigre et/ou prévenir et/ou limiter le catabolisme musculaire et la dénutrition notamment protéique.

La protéine HIP/PAP ou l'un de ses dérivés pourra également être utilisée sous forme d'une composition comprenant une quantité efficace de ladite protéine ou de l'un de ses dérivés et au moins un excipient physiologiquement acceptable.

Selon certains modes de réalisation de l'invention, la protéine HIP/PAP ou l'un de ses dérivés pourra être administrée en combinaison avec au moins un autre composé thérapeutique pour le traitement de l'insulino-résistance ou de troubles associés.

Le texte décrit également une méthode de traitement de l'insulino-résistance chez des sujets non insulino-requérants, comprenant l'administration de la protéine HIP/PAP ou l'un de ses dérivés.

Le texte décrit enfin l'utilisation de la protéine HIP/PAP ou l'un de ses dérivés pour la fabrication d'un médicament pour le traitement de l'insulino-résistance chez des sujets non insulino-requérants.

### Protéine HIP/PAP et dérivés selon l'invention :

La protéine HIP/PAP selon l'invention consiste en la protéine de séquence SEQ ID N°4.

Un dérivé de la protéine HIP/PAP décrit dans le présent texte comprend une protéine comprenant ou consistant en la séquence d'acides aminés SEQ ID N°1. La séquence d'acides aminés SEQ ID N°1 correspond à la protéine HIP/PAP de séquence SEQ ID N°4, amputée du peptide signal de 26 acides aminés en N-terminal de ladite protéine.

Selon un autre mode de réalisation décrit dans le présent texte, un dérivé de la protéine HIP/PAP comprend la séquence d'acides aminés SEQ ID N°2 ou consiste en la séquence d'acides aminés SEQ ID N°2. La séquence d'acides aminés SEQ ID N°2 correspond à la forme courte de la protéine HIP/PAP et, comparée à la séquence d'acide aminés SEQ ID N°1, est amputée du pro-peptide de 11 acides aminés en position N-terminale.

Dans un mode de réalisation alternatif, un dérivé de la protéine HIP/PAP comprend ou consiste en la séquence SEQ ID N°3. La séquence SEQ ID N°3 correspond à la séquence SEQ ID N°1, à laquelle une méthionine a été ajoutée en position N-terminale. Le dérivé HIP/PAP de séquence SEQ ID N°3 est plus particulièrement illustré dans les exemples et est également appelé rcHIP/PAP ou ALF5755. Ce dérivé peut notamment être produit de façon recombinante dans des cellules de *E.Coli.* Le propeptide N-terminal de 12 acides aminés (propeptide de 11 acides aminés plus la méthionine additionnelle) peut être clivé, afin d'obtenir la forme courte de la protéine HIP/PAP (SEQ ID N°2).
Les formes courte ou longue de la protéine HIP/PAP ou de ses dérivés peuvent être utilisées indifféremment.

Par dérivé de la protéine HIP/PAP, il est également entendu les formes dérivées biologiquement actives de la protéine HIP/PAP de séquence SEQ ID N°4 ou des formes amputée du peptide signal ou courte, respectivement de séquences SEQ ID N°1 ou 2. Par biologiquement actif, on entend que les dérivés de la protéine HIP/PAP possèdent la même activité biologique, que la protéine HIP/PAP, ou que les formes de séquences SEQ ID N°1 ou 2. A titre d'exemple, un dérivé biologiquement actif de la protéine HIP/PAP possède, lorsqu'il est administré en quantité efficace (voir les protocoles définis dans la partie expérimentale) l'une au moins des activités suivantes :
- Diminution de l'indice HOMA-IR pendant le test OGTT chez des souris ob/ob ou ayant suivi un régime hyperlipidique (High Fat Diet), tel que défini dans les exemples.
- Diminution de la glycémie lors du test de tolérance au glucose, vis-à-vis d'animaux contrôles.
- Diminution de la masse grasse et/ou augmentation de la masse maigre.
- Augmentation de l'absorption musculaire du glucose.

L'invention concerne les dérivés de la protéine HIP/PAP selon l'invention correspondant à une protéine ayant au moins 90 % d'identité avec une protéine choisie parmi le groupe formé par les séquences d'acides aminés de SEQ ID 1 à 4.
Il est entendu qu'une protéine ayant au moins 90 % d'identité avec une protéine de référence possédera au moins 91%, 92%, 93%, 94%, 95%, 96%, 97%, 98% or 99% d'identité en acides aminés avec ladite protéine de référence.
Afin de déterminer le pourcentage d'identité de deux séquences d'acides aminés pour les besoins de l'invention, les séquences seront alignées pour permettre une comparaison optimale. Des espaces (gap) peuvent être introduits dans l'une ou l'autre des séquences à aligner afin de permettre un alignement optimal, et les séquences non homologues peuvent être ignorées pour la comparaison. Le pourcentage d'identité des deux séquences d'acides aminés comparées peut être obtenu comme décrit dans le livre de D. Voet et J.G. Voet, Biochimie (2nd Edition, De Boeck & Larcier, 2005, section 7.4, paragraph B). Les alignements sont réalisés avec le logiciel CLUSTAL W (version 1.82) avec les paramètres suivants : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

Les dérivés biologiquement actifs de la protéine HIP/PAP incluent les peptides comprenant une séquence d'acides aminés suffisamment homologue à la protéine HIP/PAP ou à l'une des séquences d'acides aminés SEQ ID N°1 à 4, qui comprennent le même nombre d'acides aminés que la séquence de référence correspondant et qui possèdent la même activité biologique.
Les dérivés biologiquement actifs de la protéine HIP/PAP incluent également les peptides comprenant une séquence d'acides aminés suffisamment homologue à la protéine HIP/PAP ou à l'une des séquences d'acides aminés SEQ ID N°1 à 4, qui comprennent un nombre supérieur d'acides aminés à la séquence de référence correspondant et qui possèdent la même activité biologique.

En plus des variants alléliques naturels de la portion biologiquement active de la protéine HIP/PAP qui existent chez le mammifère, l'homme du métier comprendra que des changements supplémentaires peuvent être introduits par des mutations dans les séquences SEQ ID N°1 à 4, qui n'altèrent pas l'activité biologique desdits variants. En particulier des substitutions d'acides aminés non essentiels peuvent être apportées dans les séquences correspondant à la protéine HIP/PAP ou aux dérivés de séquences SEQ ID N°1 à 3. Un acide aminé « non essentiel » est un acide aminé, dont le changement vis-à-vis de la séquence de référence, et en particulier, vis-à-vis de la protéine HIP/PAP sauvage (de séquence SEQ ID N°4) n'altère pas l'activité biologique. Par opposition, un acide aminé « essentiel » consiste en un acide aminé dont le changement altère l'activité biologique de la protéine ou du peptide dérivé.

Dans certains modes de réalisation, la protéine HIP/PAP, ou l'un de ses dérivés, peut être associée ou combinée par des liaisons non covalentes avec des portions non-HIP/PAP. Par exemple, la protéine HIP/PAP ou l'un de ses dérivés pourrait être associée à une particule de liposome. En fonction du type de liposome, ou du procédé de fabrication, la protéine HIP/PAP, ou son dérivé, pourra être associée en surface du liposome ou encapsulée à l'intérieur dudit liposome.

La protéine HIP/PAP, ou l'un de ses dérivés, pourrait également être associée à des portions non-HIP/PAP par des liaisons covalentes. De telles portions non-HIP/PAP peuvent être sélectionnées parmi les composés protéiques ou non, par exemple, le polyéthylène glycol, formant ainsi un dérivé HIP/PAP pégylé.
Un dérivé de la protéine HIP/PAP selon l'invention comprend également les dérivés qui ne deviennent biologiquement actifs que lors de leur administration au patient.
Enfin, les dérivés de la protéine HIP/PAP comprennent également les protéines chimériques ou les protéines de fusion. De telles protéines sont fusionnées avec des polypeptides non-HIP/PAP. Ces derniers pouvant être fusionnés avec la partie N ou C-terminale. Typiquement la protéine HIP/PAP ou l'un de ses dérivés peuvent être fusionnés avec la séquence GST au niveau de leur partie C-terminale, afin de faciliter la purification des protéines recombinantes.

Dans certains modes de réalisation, la protéine HIP/PAP, ou l'un de ses dérivés, est produit de façon recombinante dans des cellules bactériennes ou animales, incluant les cellules d'insectes et de mammifères, selon des techniques connues de l'homme du métier.
Dans d'autres modes de réalisation, la protéine HIP/PAP, ou l'un de ses dérives, tel que décrit ci-dessus peut être isolée à partir d'une cellule ou d'un tissu par des techniques connues de purification.
La protéine HIP/PAP et ses dérivés peuvent également être produits par synthèse chimique.
Dans la suite du texte, le terme protéine HIP/PAP recouvrera la protéine HIP/PAP en elle-même, ainsi que ses dérivés tels que décrits ci-dessus.

### Compositions selon l'invention :

L'invention se rapporte également à une composition comprenant la protéine HIP/PAP ou l'un de ses dérivés tels que décrits précédemment, pour son utilisation pour traiter, réduire, limiter ou prévenir le développement de l'insulino-résistance chez des patients non insulino-requérants.
Une telle composition pourra également être utilisée pour augmenter le ratio masse maigre / masse grasse, chez des patients présentant des troubles du métabolisme énergétique.
Selon différents modes de réalisation, l'invention est particulièrement avantageuse pour le traitement de sujets tels que définis précédemment.

Une telle composition comprendra une quantité efficace de la protéine HIP/PAP et au moins un excipient physiologiquement acceptable, en particulier un excipient pharmaceutiquement acceptable. Par excipient physiologiquement acceptable, on entend un excipient non toxique pour le sujet auquel il est administré aux dosages et concentrations employés. Les excipients pharmaceutiquement acceptables correspondent aux excipients classiquement utilisés par l'homme du métier dans le cadre de préparation pharmaceutique. Les excipients sont choisis suivant la forme pharmaceutique et le mode d'administration souhaité parmi les excipients habituels qui sont connus de l'homme du métier (voir Remington's Pharmaceutical Sciences, 16ème édition, Osol, A éd., 1980).
A titre d'exemple, des compositions décrites dans le présent texte pourront comprendre, en fonction des indications thérapeutiques et de la protéine HIP/PAP ou de son dérivé :
a) la protéine HIP/PAP ou l'un de ses dérivés ;
b) un tampon capable de maintenir le pH dans une gamme de stabilité maximale, préférentiellement de 1 à 9, plus particulièrement de 4 à 8 et plus particulièrement encore de 6 à 7,5 ;
c) un détergent ou un surfactant stabilisant la protéine ou le polypeptide contre l'agrégation induite par l'agitation
d) un isotonique
e) un conservateur, choisi par exemple parmi le groupe constitué par les phénols, les benzyl alcools, les benzothélium halides, les chlorides
f) de l'eau
Si le détergent ou le surfactant employé est non-ionique, il pourra être choisi parmi les polysorbates, le PLURONIC TM, le polyéthylène glycol (PEG) ou les poloxamères.
Un isotonique permettra de maintenir l'isotonicité de la composition et inclura typiquement des polyalcools comme la glycérine, l'érythritol, l'arabitol, le xylitol, le sorbitol ou le mannitol, utilisés seuls ou en combinaison. Alternativement, le chlorure de sodium et/ou tout autre sel inorganique, pourra être utilisé en tant qu'isotonique.
Le tampon pourra être par exemple, l'acétate, le citrate, le succinate, un tampon phosphate ou tout autre tampon inorganique, en fonction du pH désiré.
Les conservateurs de types phénol, benzyl alcool, benzothélium halides et chlorides sont des agents anti-microbiens connus. Des conservateurs typiques comprennent l'octadécyldiméthylbenzyl ammonium chloride, l'hexaméthonium chloride, le benzalkonium chloride, les phénol, butyl ou benzyl alcools, les alkyl parabènes comme le méthyl ou propyl parabène, le catéchol, le résorcinol, le cyclohexanol, le 3-pentanol et le m-crésol.
Des excipients complémentaires pourront aussi comprendre, les antioxydants, comme l'acide ascorbique et la méthionine, des agents chélateurs, comme l'EDTA, des sucres comme le sucrose, le mannitol, le tréhalose ou le sorbitol, etc.

La protéine HIP/PAP ou son dérivé pourra être sous forme d'un sel pharmaceutiquement acceptable. On entend ainsi des sels préparés à partir d'acides non toxiques ou de bases non toxiques, pharmaceutiquement acceptables, incluant les sels et acides organiques et inorganiques. A titre d'exemple, on peut citer les sels de métaux alcalins (sels de sodium et de potassium), les sels de métaux alcalino-terreux (sels de calcium et de magnésium), les sels d'ammonium, les sels de bases organiques (sels de pyridine ou de triéthylamine), les sels d'acides inorganiques (hydrochloride, sulfate, nitrate) et les sels d'acides organiques (acétate, oxalate, p-toluènesulfonate).

### Modes d'administration de la protéine HIP/PAP :

Selon un mode de réalisation préféré, la protéine HIP/PAP est administrée dans une quantité efficace, c'est-à-dire la quantité nécessaire pour obtenir les effets attendus de l'invention. Une telle quantité de protéine HIP/PAP sera déterminée, généralement de façon empirique, en fonction de la condition pathologique visée et du sujet à traiter. La quantité efficace dépendra également du mode d'administration envisagé, du composé administré (protéine HIP/PAP ou dérivé) et de sa formulation. Les ajustements nécessaires à la détermination de la quantité efficace pour obtenir l'effet thérapeutique maximal correspondent à des techniques de routine pour le clinicien.
Partant des résultats illustrés dans les exemples, une quantité efficace de la protéine HIP/PAP est comprise entre 0,1 µg/jour/kg de poids corporel et environ 100 mg/jour/kg de poids corporel. Bien que dans certains modes de réalisation, une quantité efficace de la protéine HIP/PAP puisse atteindre plus de 10 mg/kg, une quantité efficace de la protéine HIP/PAP selon la présente invention est généralement inférieure à 5 mg/kg de poids corporel, ce qui inclut des quantités inférieures à 4,5 mg/kg, 4 mg/kg, 3,5 mg/kg, 3 mg/kg, 2,5 mg/kg ou 2000 µg/kg. Plus particulièrement, une quantité efficace de la protéine HIP/PAP selon la présente invention comprend des quantités d'au moins 1 µg/kg, 2 µg/kg, 3 µg/kg, 4 µg/kg, 5 µg/kg, 6 µg/kg, 7 µg/kg, 8 µg/kg, 9 µg/kg, 10 µg/kg, 15 µg/kg, 20 µg/kg, 25, µg/kg, 30 µg/kg, 40 µg/kg, 50 µg/kg, 60 µg/kg, 70 µg/kg, 80 µg/kg, 90 µg/kg, 100 µg/kg, 150 µg/kg, 200 µg/kg, 250 µg/kg, 300 µg/kg, 350 µg/kg, 400 µg/kg, 450 µg/kg, 500 µg/kg, 600 µg/kg, 700 µg/kg, 800 µg/kg, 900 µg/kg, 1 mg/kg, 2 mg/kg, 3 mg/kg, 4 mg/kg, 5 mg/kg ou plus vis-à-vis du poids corporel.
Selon des modes particuliers de réalisation, la protéine HIP/PAP est administrée selon un dosage compris entre 10 et 5000 µg/kg, préférentiellement entre 100 et 2000 µg/kg.
Chez la souris, une dose efficace typique déterminée expérimentalement est comprise entre 10 et 2000 µg/kg de poids corporel, plus particulièrement entre 150 et 1500 µg/kg de poids corporel. Les adaptations inter-espèces de dosage peuvent être réalisée selon une méthode connue de l'état de la technique, par exemple comme décrit dans l'article de Mordenti et al., Pharmaceut. Res. 8, p. 1351 (1991). Classiquement chez la souris adulte de poids normal, la dose efficace est comprise entre 1 et 100 µg/jour.
Classiquement, chez l'homme, la dose efficace de la protéine HIP/PAP débute à environ 3 mg chez des patients pesant approximativement 70 kg, soit approximativement 40 µg/kg de poids corporel.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif (la protéine HIP/PAP ou l'un de ses dérivés) peut être administré sous forme unitaire d'administration, en mélange avec des excipients pharmaceutiques, tels que décrits ci-dessus et/ou classiques des animaux et des êtres humains pour la prévention ou le traitement de l'insulino-résistance.
Les modes d'administration préférés sont les voies entérale, en particulier orale, et intraveineuse. A titre d'exemple, une composition de l'invention pourra être administrée par voie intraveineuse en continue ou par bolus, ou quotidiennement par voie orale.

Avantageusement, un produit de combinaison selon l'invention est administré par voie orale. Les formes appropriées pour la voie orale sont par exemple les comprimés, les gélules, les pastilles, les poudres, les granulés, les lyophilisats, les solutés buvables et les sirops. Les comprimés, poudres, les granulés, les lyophilisats, les solutés buvables et les sirops constituent la forme pharmaceutique ou cosmétique adaptée à la voie orale actuellement préférée. Les comprimés ou gélules peuvent être de nature variée, à libération immédiate, contrôlée ou retardée et éventuellement sous forme effervescente ou orodispersible. On obtient une préparation en gélule en mélangeant l'ingrédient actif (i) ou (ii) avec un diluant et en versant le mélange obtenu dans des gélules molles ou dures.
Les formes pour l'administration orale, telle que les gélules ou comprimés sont un mode de réalisation de l'invention avantageux. Plus particulièrement les formes unitaires d'administration de la protéine HIP/PAP, comme les gélules, comprimés, sachets ou ampoules pour suspension buvable, comprendront typiquement des quantités de protéine HIP/PAP ou de l'un quelconque de ses dérivés allant de 0,1 à 200 mg et plus particulièrement de 50 à 100 mg.

Une préparation sous forme de sirop ou d'élixir peut par exemple contenir HIP/PAP conjointement avec un édulcorant, un antiseptique, un conservateur, un agent donnant du goût ou un colorant approprié.
Les poudres, lyophilisats ou les granules dispersibles dans l'eau peuvent contenir HIP/PAP en mélange avec des agents de dispersion ou des agents mouillants ou des agents de mise en suspension, de même qu'avec des correcteurs de goûts ou des édulcorants.

Selon l'invention, la protéine HIP/PAP, sous forme adaptée à la prise par voie orale, peut être également destinée à une utilisation en tant que complément alimentaire. Une telle préparation est particulièrement adaptée aux utilisations dans le cadre de la prévention de l'insulino-résistance chez des sujets à risque et/ou chez des patients dénutris, présentant un état hyper-catabolique, ou présentant une sarcopénie. Une telle préparation est également adaptée à une utilisation de HIP/PAP visant à augmenter le ratio masse maigre / masse grasse des individus.

La protéine HIP/PAP pourra être stérilisée préalablement à l'administration *in vivo.* La stérilisation pourra être obtenue par filtration sur des membranes de filtration stériles, avant ou après la lyophilisation ou la reconstitution. La protéine HIP/PAP administrée de façon systémique pourra avantageusement être lyophilisée ou stockée en solution. Sous forme lyophilisée, la protéine HIP/PAP est généralement formulée en combinaison avec des excipients permettant la reconstitution avec un diluant approprié, lors de l'utilisation.

La protéine HIP/PAP pourra être administrée quotidiennement en une prise ou de façon fractionnée (par exemple de 2 à 3 fois par jour) jusqu'à obtention de l'effet thérapeutique désiré. Elle pourra également être administrée de façon chronique, de façon à prévenir ou limiter le développement de la résistance à l'insuline, par exemple chez des sujets à risque, présentant des conditions physiologiques et/ou pathologiques favorables au développement d'une insulino-résistance.

La protéine HIP/PAP pourra également être administrée sous forme de cure, par exemple des cures allant de 15 jours à 3 mois, éventuellement répétées de 1 à 6 fois à des doses et des intervalles de temps déterminés. Ces modes d'administration seront particulièrement indiqués dans le cadre de la prévention de l'insulino-résistance chez des sujets à risque et/ou chez des patients dénutris, présentant un état hyper-catabolique, ou présentant une sarcopénie.

La protéine HIP/PAP selon l'invention pourra également être combinée dans le cadre d'une poly-thérapie avec d'autres composés pour le traitement de l'insulino-résistance ou des conditions associées, comme les dyslipidémies ou l'athérosclérose.

La protéine HIP/PAP selon l'invention pourra également être administrée en combinaison avec des mesures hygiéno-diététiques, comme un régime hypocalorique et/ou hypolipidique et une augmentation de l'activité physique.

### Utilisation selon l'invention :

La présente invention se rapporte également à l'utilisation de la protéine HIP/PAP ou de l'un de ses dérivés, tels que décrits précédemment, pour traiter, réduire, limiter ou prévenir le développement de l'insulino-résistance, chez les sujets non insulino-requérants, tels que définis dans les différents modes de réalisation décrits précédemment.
Selon des modes de réalisation particuliers de l'invention, la protéine HIP/PAP sera utilisée chez les sujets tels que décrits précédemment.
Dans certains modes de réalisation de l'invention, le dérivé de la protéine HIP/PAP sera caractérisé en ce qu'il comprend une séquence en acide aminé ayant une identité de séquence d'au moins 90 % avec le polypeptide constitué de la séquence d'acide aminés choisi parmi les séquences SEQ ID N°1 ou 2.

### FIGURES

Figure 1 : Effet du traitement ALF5755 (■) vs placebo (●) sur la résistance à l'insuline. Fig. 1A : évolution de la glycémie (mg/dL) pendant le test de tolérance à l'insuline (ITT) chez les souris ob/ob après 2 semaines de traitement. Fig. 1B-C : évolution de l'insulinémie (ng/mL) pendant l'OGTT (B), et de l'indice HOMA-IR (C) chez les souris HFD après 4 semaines de traitement.
Fig.1D : captation musculaire du glucose chez les souris traitées 4 semaines par ALF5755 (barre blanche) ou placebo (barre noire).
Figure 2. A : Effet du traitement ALF5755 vs placebo sur la masse maigre (Fig. 2B et 2D) et la masse grasse (Fig. 2A et 2C) les souris ayant suivi un régime HFD (2A, 2C) et contrôle (2B, 2D). Fig. 2E-F : rapport [masse grasse / masse maigre] chez les souris ayant suivi un régime HFD (2E) ou contrôle (2F).
Figure 3. Effet du traitement ALF5755 (barre blanche) vs placebo (barre noire) sur le profil lipidique sérique chez les souris ayant suivi un régime HFD (3A) ou contrôle (3B). Fig.3C : bilan hépatique sérique chez les souris ayant suivi un régime HFD.

### EXEMPLES

### MATERIELS ET METHODES :

### Modèles animaux

2 modèles distincts d'insulino-résistance et de diabète ont été utilisés, le modèle ob/ob et le modèle High Fat Diet (HFD).

Le modèle ob/ob est un modèle génétique du diabète de type II dû à une mutation non-sens dans le gène de la leptine résultant en une absence de leptine chez ces animaux. Cette souris présente un syndrome d'insulino-résistance avec hyperinsulinémie, obésité, hyperglycémie et hyperlipidémie (Pelleymounter MA, Cullen MJ, Baker MB, Hecht R, Winters D, Boone T, et al. Effects of the obese gene product on body weight régulation in ob/ob mice. Science 1995 ; 269 : 540-3). Les souris ob/ob présentent une hyperglycémie (248 +/- 14 mg/dL) ainsi qu'une hyperinsulinémie majeure (9,5 +/- 0,9 ng/mL), comme classiquement décrit.

Le modèle HFD est un modèle de diabète de type II dû à un enrichissement en lipides du régime alimentaire. Dans un régime classique (control diet = CTD) l'apport calorique est de 3200 kcal/kg (21,4 % protéines, 5,1 % lipides, 47,1 % carbohydrates). Pour le régime HFD, l'apport calorique est de 4655 kcal/kg (17 % protéines, 27,5 % lipides, 37,5 % carbohydrates). Le poids des animaux HFD augmente graduellement pour devenir significativement différent de celui des animaux soumis au régime contrôle à partir de 4 semaines. Après 10 semaines de HFD, le poids moyen des animaux est supérieur d'environ 30 % à celui des animaux sous régime contrôle. Les souris HFD présentent un syndrome d'insulino-résistance avec hyperinsulinémie, obésité, hyperglycémie et hyperlipidémie (Migrenne S, Lacombe A, Lefèvre AL, Pruniaux MP, Guillot E, Galzin AM, Magnan C. Adiponectin is required to mediate rimonabant-induced improvement of insulin sensitivity but not body weight loss in diet-induced obese mice. Am J Physiol Regul Integr Comp Physiol. 2009 296(4):929-35.).
Dix semaines de régime HFD entrainent une augmentation significative de la glycémie basale (220,6 ± 3,7 mg/dL; one-tailed t-test *P* = 0,0001, ****) et de l'insulinémie basale (0,51 ± 0,06 ng/mL; One-tailed t-test *P* = 0,0069, **) comparées au régime contrôle.
Les souris soumises à un régime normal de contrôle présentent une glycémie (190,4 ± 6,9 mg/dL) et une insulinémie (0,29 ± 0,04 ng/mL) basales et au cours du test OGTT qui sont normales, et non impactées par l'administration d'ALF-5755 entre les semaines 10 et 14. Pour toutes les expérimentations, les souris ont été acclimatées à l'animalerie en respectant un cycle nuit-jour comprenant 12h d'éclairage (7h-19h).

### Traitements

### Modèle Ob/Ob :

29 animaux ont été répartis en 3 groupes : 10 animaux ont reçu du placebo (sérum physiologique), 9 animaux ont reçu une pompe délivrant 9 µg/jour d'ALF5755 et 10 animaux ont reçu 43 µg/jour d'ALF5755, pendant 4 semaines. La délivrance se fait par diffusion à partir d'une pompe Alzet (Alzet #2004) implantée en sous-cutané dans le dos de l'animal et délivrant un volume constant en sous-cutané au débit de 0,25 µL/heure. Le traitement passe dans la circulation sanguine et permet d'obtenir une concentration sérique moyenne d'ALF5755 (hipémie) nulle pour le groupe placebo, de 102,7 ± 28,6 ng/mL pour le groupe recevant 9 µg/jour d'ALF5755 et de 229,5 ± 44,9 ng/mL pour le groupe recevant 43 µg/jour d'ALF5755. Pour 3 animaux du groupe ALF5755 9 µg/jour et 3 animaux du groupe 43 µg/jour la délivrance d'ALF5755 ne s'est pas faite et l'hipémie était nulle au terme du traitement. Ces animaux ont été exclus des analyses. Deux animaux sont décédés pendant l'expérience, une souris du groupe contrôle et une souris du groupe ALF5755 43µg/jour. Les effectifs retenus pour chaque groupe sont donc : placebo, n = 9 ; ALF5755 9 µg/jour, n = 6 ; ALF5755 43 µg/jour, n = 6.

### Modèle HFD

Les animaux sous HFD et sous régime contrôle sont traités par un placebo (sérum physiologique, n = 5 pour le groupe CTD, n = 9 pour le groupe HFD) ou ALF5755 à 43 µg/jour (n = 5 pour le groupe CTD, n = 9 pour le groupe HFD) pendant 4 semaines. La délivrance se fait par diffusion à partir d'une pompe Alzet (Alzet #2004) implantée en sous-cutané dans le dos de l'animal et délivrant un volume constant en sous-cutané au débit de 0,25 µL/heure. Le traitement passe dans la circulation sanguine et permet d'obtenir une concentration sérique moyenne d'ALF5755 (hipémie) nulle pour le groupe placebo et de 309 ng/mL à J17 pour les 2 groupes recevant 43 µg/jour d'ALF5755. A J27 (dernier jour de délivrance par la pompe) les hipémies sont de 369,3 ng/mL pour les animaux du groupe CTD et 334,8 ng/mL pour le groupe HFD confirmant ainsi la bonne délivrance d'ALF5755 tout au long du traitement.

### Modèle HFD pour sensibilité tissulaire (glucose marqué au Carbone 14)

Les animaux sous HFD et sous régime contrôle sont traités par un placebo (sérum physiologique, n = 4 pour le groupe CTD, n = 6 pour le groupe HFD) ou ALF5755 à 43 µg/jour (n = 4 pour le groupe CD, n = 6 pour le groupe HFD) pendant 4 semaines, selon le même traitement que décrit précédemment. La concentration sérique moyenne d'ALF5755 (hipémie) est nulle pour le groupe placebo, de 673 ng/mL pour les animaux du groupe CTD et 784 ng/mL pour le groupe HFD confirmant la bonne délivrance d'ALF5755 tout au long du traitement.

### Variables étudiées

### Poids

Les animaux de l'expérience HDF sont pesés toutes les semaines pendant la mise en place du modèle, puis également pendant les 4 semaines de traitement pour valider la prise de poids liée au régime et évaluer un éventuel effet d'ALF5755 sur cette variable.

Les animaux ob/ob sont quant à eux pesés environ 1 fois par semaine pendant les 4 semaines de traitement.

### Prise alimentaire pour l'expérience HFD

La prise alimentaire est mesurée par pesée de l'apport et du reliquat 3 fois par semaine. Elle est ensuite rapportée à une prise quotidienne (en g/jour).

### Apport calorique pour l'expérience HFD

L'apport calorique est directement calculé à partir de la quantité de nourriture ingérée, en g, et de la valeur énergétique par gramme de croquette selon le régime.

### Glycémie basale

Les animaux sont mis à jeun 18 heures avant les différentes mesures. La glycémie basale est mesurée à l'aide d'un lecteur et de bandelettes réactives pour la mesure de la glycémie dans le sang (lecteur Glucofix® mio et bandelettes Glucofix® sensor, A. Menarini diagnostics). Une goutte de sang est suffisante pour cette mesure, elle est obtenue par prélèvement de sang de la pointe de la queue. La glycémie basale est prise avant l'implantation des pompes, après environ 2 semaines de traitement puis en fin de traitement.

### Insulinémie basale

Après la mesure de glycémie basale, un prélèvement de 75 µL de sang maximum est effectué à l'aide de capillaires pour hématocrite héparinés au sodium 3,75UI/capillaire (Hirschmann Laborgeräte) afin de doser l'insulinémie basale. Les tubes de sang sont centrifugés 3 minutes à 14000 rpm, puis le surnageant est prélevé et stocké à -20°C en vue du dosage d'insuline par ELISA (Ultra Sensitive Mouse Insulin ELISA Kit, Crystal Chem Inc, #90080) selon les recommandations d'emploi du fabricant.

### Test de tolérance au glucose administré par voie orale : OGTT

Après 18 heures de jeûne, les animaux sont pesés puis la glycémie et l'insulinémie basales sont mesurées. Une solution de glucose à 30% (CDM Lavoisier, flacon injectable 1L) est ensuite administrée par gavage intra-gastrique à raison de 2g/kg de souris.

La glycémie est mesurée au cours d'une cinétique définie (entre 5 et 90 ou 120 minutes après le gavage de glucose) de la même façon que la glycémie basale. 75 µL de sang sont également collectés à 15 et 30 minutes afin de doser l'insulinémie avec une procédure identique à celle utilisée pour le dosage de l'insulinémie basale.

Un calcul de l'indice HOMA-IR est effectué à partir des valeurs de glycémie et d'insulinémie à T0 préalablement à l'administration de glucose, 15 minutes après et 30 minutes après. L'indice HOMA-IR est calculé suivant la formule suivante :
[(Insulinémie_{(mU/L)} X Glycémie)/22,5] si la glycémie est exprimée en unité de molarité (mmol/L) ou [(Insulinémie_{(mU/L)} X Glycémie)/405] si la glycémie est exprimée en unité de masse (mg/dL).

### Test de tolérance à l'insuline administrée en sous-cutané : ITT

Les souris ob/ob sont mises à jeun pour une durée de 18h.

Une solution d'insuline diluée à 0,15UI/mL dans du sérum physiologique, à partir d'une solution mère à 100UI/mL (Novorapid Flexpen 100UI/mL, NovoNordisk A/S), est injectée par voie sous-cutanée à raison de 0,75UI/kg de souris.

La glycémie est mesurée au cours d'une cinétique définie (entre 5 et 120 minutes après l'injection d'insuline) de la même façon que précédemment décrit.

### Sensibilité tissulaire au glucose

Afin de connaître l'impact d'ALF5755 sur la sensibilité à l'insuline de différents tissus, on injecte 8 µCi de 2-déoxyglucose ¹⁴C (2DG ¹⁴C), par voie intrapéritonéale. Il s'agit d'un analogue du glucose qui n'est pas métabolisé (il ne subit que l'étape de phosphorylation par la glucokinase) ce qui lui permet de s'accumuler dans les tissus. Vingt-cinq µL de sang sont prélevés à t0, puis toutes les 10 minutes pendant 60 minutes minimum, afin d'estimer la décroissance du 2DG dans le sang. Les souris sont sacrifiées par injection létale de pentobarbital puis les tissus sont prélevés pour comptage de la radioactivité du 2DG ¹⁴C capté.

Les tissus sont digérés dans de la soude à 60°C pendant 16h, puis la solution est neutralisée. Les échantillons subissent plusieurs étapes de quantification dans le but de connaitre d'une part, la quantité de 2DG non phosphorylé, et d'autre part, la quantité totale de 2DG. Par soustraction de ces deux variables (2DG total - 2DG non phosphorylé) on calcule la quantité de 2DG qui a effectivement pénétré dans le tissu.

Cette valeur, ramenée au poids du tissu, est ensuite divisée par l'intégrale de l'activité spécifique du ¹⁴C dans le sang au cours du temps (en cpm/mg de glucose) afin de tenir compte de la quantité circulante dans le sang.

Cette méthode permet de mettre en évidence l'effet spécifique de l'insuline sur le transport de glucose dans un organe, reflet de la sensibilité à l'insuline de ce tissu.

### IRM pour détermination de la masse grasse / masse maigre :

Le scan IRM consiste à peser la souris, puis à l'introduire dans un tube et maintenir une contention serrée à l'aide d'un poussoir. Le tube est inséré dans le scanner EchoMRI, au préalable calibré avec un tube contrôle spécifique souris contenant un volume défini par le fournisseur. En 3 minutes la masse grasse, la masse maigre, les liquides biologiques et l'eau libre totale du corps sont quantifiés.

### Bilan hépatique (ALAT, ASAT, Créatinine, Bilirubine totale) :

Les bilans hépatiques sériques sont réalisés par test de coloration photométrique à l'aide de l'automate Olympus AU 400.

### Bilan lipidique (Cholestérol, Cholestérol ester, Triglycérides, Lipides neutres) :

Les bilans lipidiques sérique et hépatique sont réalisés par chromatographie HPLC.

### RESULTATS :

### Effet de la protéine HIP/PAP sur l'homéostasie glucidique :

L'administration de HIP/PAP diminue significativement la glycémie basale (après 18 heures de jeûne) chez la souris HFD (diminution de 15 % environ au 25^{ème} jour de traitement ; 188,9 ± 10,8 chez la souris traitée vs. 222,4 ± 8,5 chez la souris placebo, P < 0,001, ** ; ANOVA bidirectionnelle, effet traitement : *F*_{(16,1)}=12,76, *P* = 0,0025, **) et chez la souris ob/ob, vis-à-vis de souris placebo (diminution de 28 % environ au 25^{ème} jour de traitement ; 228,2 ± 14,7 chez la souris traitée, *vs*. 316,3 ± 32,2 chez la souris placebo, *P* < 0,05, *).

De même, le traitement avec la protéine HIP/PAP permet également de réduire l'hyperglycémie durant le test OGTT, de manière significative pour le modèle ob/ob dès 2 semaines (ANOVA bidirectionnelle, effet traitement: *F*_{(133,1)}=13,09, *P* = 0,0018, **) et maintenue à 4 semaines (ANOVA bidirectionnelle, effet traitement : *F*_{(133,1)}=5,78, *P* = 0,027, *). Pour les souris soumises au régime HFD et traitées avec HIP/PAP, on observe une tendance à l'amélioration, qui ramène la courbe d'hyperglycémie à un niveau comparable à celle observée chez les souris ayant suivi le régime contrôle.

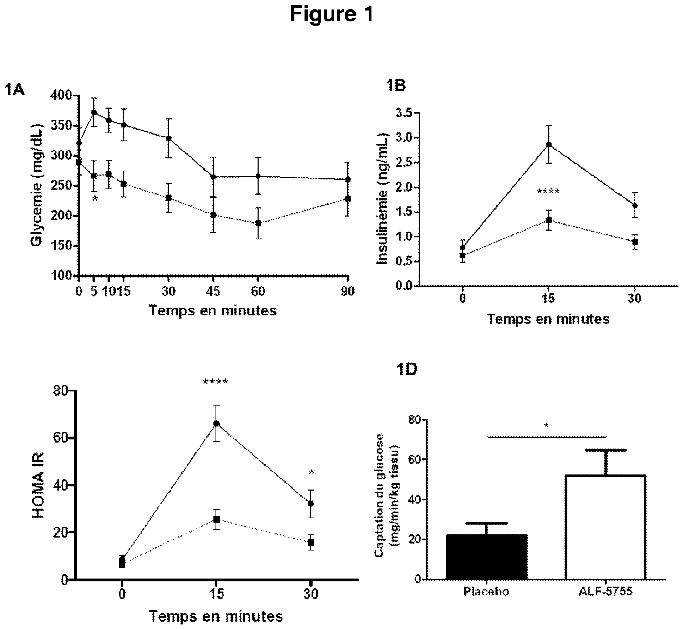

Le traitement avec la protéine HIP/PAP permet également de réduire l'hyperglycémie durant le test ITT, de manière significative pour le modèle ob/ob à 2 semaines (ANOVA bidirectionnelle, effet traitement: *F*_{(133,1)}=4,71, *P* = 0,043, *, voir fig. 1A) avec une tendance maintenue à 4 semaines traduisant une amélioration significative et spécifique de la sensibilité à l'insuline. En effet, le test ITT consiste à faire baisser la glycémie en injectant de l'insuline exogène. Pour une même dose d'insuline, plus la glycémie baisse en réponse, plus la sensibilité à l'insuline est importante (et par opposition, plus la résistance à l'insuline est faible). Comme illustré dans la figure 1A, l'injection d'insuline ne diminue quasiment pas la glycémie (au moins pendant les 30 premières minutes) chez les souris ob/ob ayant reçu le traitement placebo. Un tel effet traduit une résistance majeure à l'insuline. A l'inverse, chez les souris ob/ob traitées avec ALF5755, l'injection d'insuline entraine une baisse importante de la glycémie, traduisant ainsi une diminution importante de la résistance à l'insuline (i.e. : une amélioration de la sensibilité à l'insuline).

### Effet de la protéine HIP/PAP sur l'insulinémie et l'indice HOMA-IR après 1 mois de traitement :

Une diminution de l'hyper-insulinémie est observée pendant le test OGTT chez les souris HFD traitées par ALF-5755 par rapport aux souris ayant reçu le traitement placebo (diminution du pic d'hyper-insulinémie à 15 minutes : 3,75 fois le taux basal chez la souris traitée, *vs*. 7,61 fois chez la souris placebo, *P* < 0,0001, ****, et à 30 minutes : 2,31 fois le taux basal chez la souris traitée, *vs*. 3,7 fois chez la souris placebo, *P* < 0,05, *); Ces modifications s'accompagnent d'une normalisation de l'indice HOMA-IR dans le modèle HFD : ANOVA bidirectionnelle, effet traitement : *F*_{(32,1)}=16,77, *P* = 0,0003, ***, voir Fig.1B-C ;
Chez la souris ob/ob on observe également une amélioration de l'hyper-insulinémie pendant l'OGTT : ANOVA bidirectionnelle, effet traitement : *F*_{(34,1)}=5,84, *P* = 0,027, *. Ces modifications s'accompagnent d'une tendance à la normalisation de l'indice HOMA-IR : diminution non statistiquement significative de l'indice à 15 et 30 minutes.

L'ensemble de ces données confirment que la normalisation de la glycémie basale et de l'excursion glycémique durant le test OGTT par le traitement par ALF5755 résulte de la réduction de l'insulino-résistance et non d'un effet insulino-sécrétagogue.

### Effet de la protéine HIP/PAP sur l'insulinorésistance tissulaire:

La modulation de la captation de glucose par les différents tissus insulino-sensibles (foie, muscles et tissus adipeux) après traitement par ALF-5755 a été mesurée dans le modèle HFD par captation de 2DG marqué au carbone 14. La figure 1D illustre que le traitement avec ALF5755 entraîne une augmentation de la captation de glucose par le muscle (muscle tibialis ; 21,7µM ± 6,3 versus 51,9µM ± 12,8 ; two-tailed Mann Whitney, *P* = 0,04, *). Aucune augmentation n'a été observée dans le foie ou dans le tissu adipeux.

### Effet de la protéine HIP/PAP sur les pourcentages de masse grasse et masse maigre :

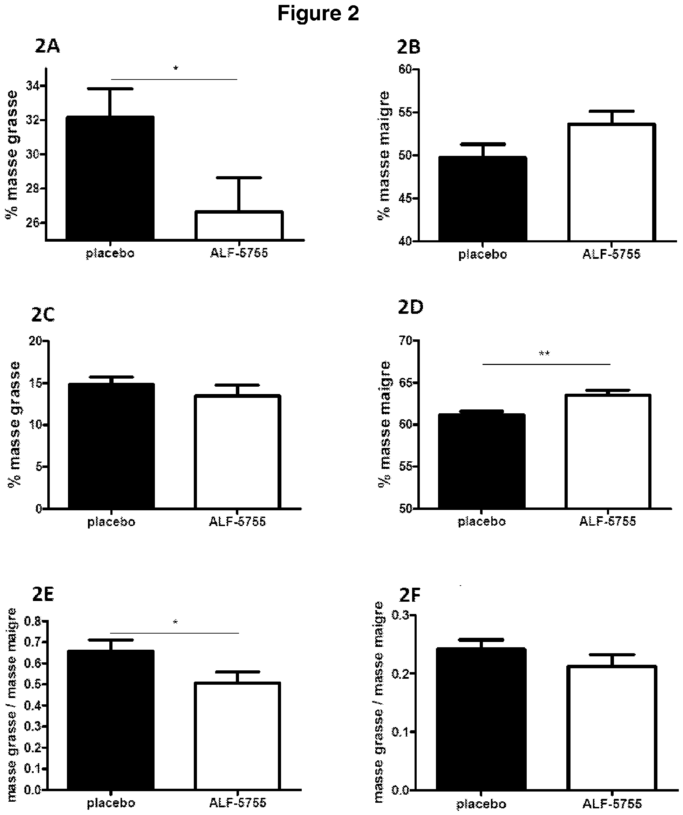

Les 14 semaines de régime HFD induisent une augmentation importante du pourcentage de masse grasse (Fig. 2A et C : 32% contre 15% dans le régime contrôle pour les groupes placébo). Le traitement avec ALF-5755 diminue de manière significative l'augmentation de masse grasse observée chez les animaux soumis au HFD (Fig. 2A : 32,1% ± 1,7% versus 26,7% ± 2,0% ; One-tailed Mann Whitney test : *P* = 0,039, *). Une tendance comparable est observée pour les souris soumises au régime contrôle. De façon similaire, on observe une diminution de la masse maigre dans le groupe d'animaux sous HFD par rapport au régime contrôle (Fig. 2B et D : 49,8% contre 61,2% dans le régime contrôle pour les groupes placébo). Le traitement avec ALF-5755 augmente de manière significative le pourcentage de masse maigre observée chez les animaux soumis au CTD (Fig. 2D : 61,2% ± 0,4% versus 63,5% ± 0,6% ; One-tailed Mann Whitney test : *P* = 0,008, **). Une tendance comparable est observée pour les souris soumises au régime HFD. L'analyse du rapport masse grasse / masse maigre montre que le traitement avec ALF-5755 entraine une diminution significative d'au moins 20 % (21 %) de ce ratio chez les souris ayant suivi le régime HFD (Mann-Whitney one-tailed test, P = 0,047) ainsi qu'une tendance à la diminution d'au moins 10 % (10,1 %) chez les souris soumises au régime contrôle, vis-à-vis des souris ayant reçu un traitement placebo.

### Effet de la protéine HIP/PAP sur les profils lipidiques sériques et hépatiques :

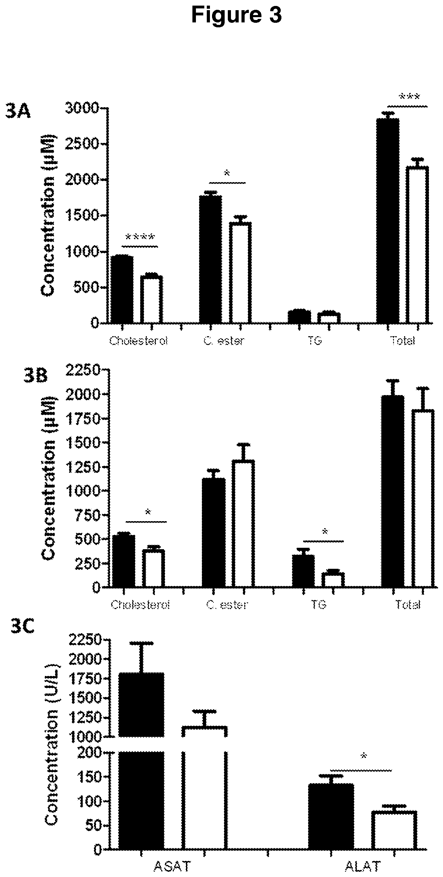

Dans les groupes d'animaux HFD (voir Fig. 3A) on observe qu'ALF-5755 entraine une diminution statistiquement significative du taux de cholestérol (913,0µM ± 28,2 versus 647,1µM ± 32,9 ; two-tailed Mann Whitney, *P* < 0,0001, ****), du taux de cholestérol esters (1760,6µM ± 65,8 versus 1390,3µM ± 96,7 ; two-tailed Mann Whitney Whitney, *P* = 0,011, *), du taux de lipides neutres total (2834,9µM ± 95,9 versus 2168,6µM ± 118,5 ; two-tailed Mann Whitney, *P* = 0,0005, ***), et une tendance à la baisse du taux de triglycérides (161,3µM ± 15,0 versus 131,2µM ± 24,1).

Des effets comparables et statistiquement significatifs sont observés avec le traitement par ALF-5755 dans le régime contrôle (Fig.3B) pour le cholestérol (526,6µM ± 35,9 versus 376,9µM ± 43,9 ; two-tailed Mann Whitney, *P* = 0,032, *), et pour les triglycérides (323,2µM ± 72,6 versus 143,0µM ± 31,5 ; two-tailed Mann Whitney, *P* = 0,016, *).

Dans le modèle ob/ob (Fig.3C) ALF-5755 diminue le cholestérol (763,0µM ± 58,5 versus 532,5µM ± 29,0 ; two-tailed Mann Whitney, *P* = 0,002, **), et on observe une tendance à la diminution du taux de triglycérides (651,7µM ± 168,6 versus 271,8µM ± 44,6).

On observe également une tendance à la diminution des ASAT et une diminution statistiquement significative des ALAT chez les souris HFD traitées par ALF-5755 (132,7µM ± 19,3 versus 77,2µM ± 12,7 ; two-tailed Mann Whitney, *P* = 0,032, *, voir Fig. 3C). De plus, des tendances comparables sont observées dans le régime contrôle et dans le modèle de souris ob/ob.

## Revendications

1. Protéine HIP/PAP, ou l'un de ses dérivés, pour son utilisation pour le traitement ou la prévention de la résistance à l'insuline chez des sujets non insulino-requérants,
**caractérisé en ce que** ledit dérivé comprend une séquence en acides aminés ayant une identité de séquence d'au moins 90 % avec le polypeptide constitué de la séquence d'acides aminés choisie parmi les séquences SEQ ID No 1 à 4.

2. Protéine HIP/PAP, ou l'un de ses dérivés, pour son utilisation selon la revendication 1 **caractérisée en ce que** les sujets présentent une valeur HOMA B, supérieure ou égale à 60 % et/ou un indice HOMA-IR inférieur à 6.

3. Protéine HIP/PAP, ou l'un de ses dérivés, pour son utilisation selon l'une quelconque des revendications 1 ou 2, **caractérisée en ce que** les sujets présentent une glycémie à jeun inférieure à 125 mg/dL, et plus particulièrement inférieure à 110 mg/dL à jeun.

4. Protéine HIP/PAP, ou l'un de ses dérivés, pour son utilisation selon l'une quelconque des revendications 1 à 3, **caractérisée en ce que** les sujets présentent une glycémie, 2h après un test de tolérance oral au glucose, inférieure à 200 mg/dL, et plus particulièrement inférieure à 140 mg/dL.

5. Protéine HIP/PAP, ou l'un de ses dérivés, pour son utilisation selon l'une quelconque des revendications précédentes, **caractérisée en ce que** les sujets ont une tolérance glucidique normale.

6. Protéine HIP/PAP, ou l'un de ses dérivés, pour son utilisation selon l'une quelconque des revendications précédentes, **caractérisée en ce que** les sujets sont normo-glycémiques à jeun.

7. Protéine HIP/PAP, ou l'un de ses dérivés, pour son utilisation selon l'une quelconque des revendications précédentes, **caractérisée en ce que** les sujets présentent au moins l'un des troubles choisi parmi le groupe constitué par : le surpoids viscéral, l'obésité, l'hyperandrogénie, un trouble de l'alimentation, la sarcopénie, l'hypercatabolisme, la dénutrition.

8. Protéine HIP/PAP, ou l'un de ses dérivés, pour son utilisation selon l'une quelconque des revendications précédentes, pour le traitement de l'insulino-résistance associée au vieillissement.

9. Protéine HIP/PAP, ou l'un de ses dérivés, pour son utilisation chez des sujets non insulino-requérants, pour augmenter le développement musculaire, stimuler la prise de masse maigre et prévenir ou limiter le catabolisme musculaire et la dénutrition protéique,
**caractérisé en ce que** ledit dérivé comprend une séquence en acides aminés ayant une identité de séquence d'au moins 90 % avec le polypeptide constitué de la séquence d'acides aminés choisie parmi les séquences SEQ ID No 1 à 4.

10. Protéine HIP/PAP, ou l'un de ses dérivés, pour son utilisation chez des sujets non insulino-requérants, pour prévenir ou traiter la dyslipidémie et en particulier l'hypercholestérolémie
**caractérisé en ce que** ledit dérivé comprend une séquence en acides aminés ayant une identité de séquence d'au moins 90 % avec le polypeptide constitué de la séquence d'acides aminés choisie parmi les séquences SEQ ID No 1 à 4.

11. Protéine HIP/PAP, ou l'un de ses dérivés, pour son utilisation chez des sujets non insulino-requérants, pour prévenir ou traiter l'athérosclérose et en particulier la maladie coronarienne et l'artériopathie des membres inférieurs,
**caractérisé en ce que** ledit dérivé comprend une séquence en acides aminés ayant une identité de séquence d'au moins 90 % avec le polypeptide constitué de la séquence d'acides aminés choisie parmi les séquences SEQ ID No 1 à 4.

12. Composition comprenant une quantité efficace de la protéine HIP/PAP ou de l'un de ses dérivés, tels que définis selon l'une quelconque des revendications 1 à 11, en mélange avec au moins un excipient physiologiquement acceptable, pour son utilisation pour traiter ou prévenir l'insulino-résistance chez le sujet non insulino-requérant.

## Patentansprüche

1. HIP/PAP-Protein oder eines seiner Derivate für die Verwendung zur Behandlung oder Vorbeugung von Insulinresistenz bei Individuen ohne Insulinbedarf, **dadurch gekennzeichnet, dass** das Derivat eine Aminosäuresequenz umfasst, die eine Sequenzidentität von mindestens 90% mit dem Polypeptid aufweist, das aus der Aminosäuresequenz, ausgewählt aus den Sequenzen SEQ ID Nr. 1 bis 4, besteht.

2. HIP/PAP-Protein oder eines seiner Derivate für die Verwendung nach Anspruch 1, **dadurch gekennzeichnet, dass** die Individuen einen HOMA-B-Wert von mehr als oder gleich 60% und/oder einen HOMA-IR-Index von weniger als 6 aufweisen.

3. HIP/PAP-Protein oder eines seiner Derivate für die Verwendung nach einem der Ansprüche 1 oder 2, **dadurch gekennzeichnet, dass** die Individuen einen Nüchternblutzuckerspiegel von weniger als 125 mg/dl und insbesondere weniger als 110 mg/dl nüchtern aufweisen.

4. HIP/PAP-Protein oder eines seiner Derivate für die Verwendung nach einem der Ansprüche 1 bis 3, **dadurch gekennzeichnet, dass** die Individuen 2 Std. nach einem oralen Glukosetoleranztest einen Blutzucker von weniger als 200 mg/dl und insbesondere weniger als 140 mg/dl aufweisen.

5. HIP/PAP-Protein oder eines seiner Derivate für die Verwendung nach einem der vorhergehenden Ansprüche, **dadurch gekennzeichnet, dass** die Individuen eine normale Kohlenhydrattoleranz aufweisen.

6. HIP/PAP-Protein oder eines seiner Derivate für die Verwendung nach einem der vorhergehenden Ansprüche, **dadurch gekennzeichnet, dass** die Individuen einen normalen Nüchternblutzuckerspiegel aufweisen.

7. HIP/PAP-Protein oder eines seiner Derivate für die Verwendung nach einem der vorhergehenden Ansprüche, **dadurch gekennzeichnet, dass** die Individuen mindestens eine der Störungen aufweisen, ausgewählt aus der Gruppe, bestehend aus: viszeralem Übergewicht, Adipositas, Hyperandrogenismus, einer Essstörung, Sarkopenie, Hyperkatabolismus, Fehlernährung.

8. HIP/PAP-Protein oder eines seiner Derivate für die Verwendung nach einem der vorhergehenden Ansprüche zur Behandlung von altersbedingter Insulinresistenz.

9. HIP/PAP-Protein oder eines seiner Derivate für die Verwendung bei Individuen ohne Insulinbedarf zur Erhöhung des Muskelaufbaus, Stimulation der Zunahme der fettfreien Masse und zur Verhinderung oder Einschränkung des Muskelkatabolismus und der Proteinmangelernährung,
**dadurch gekennzeichnet, dass** das Derivat eine Aminosäuresequenz umfasst, die eine Sequenzidentität von mindestens 90% mit dem Polypeptid aufweist, das aus der Aminosäuresequenz, ausgewählt aus den Sequenzen SEQ ID Nr. 1 bis 4, besteht.

10. HIP/PAP-Protein oder eines seiner Derivate für die Verwendung bei Individuen ohne Insulinbedarf zur Vorbeugung oder Behandlung von Dyslipidämie und insbesondere Hypercholesterinämie,
**dadurch gekennzeichnet, dass** das Derivat eine Aminosäuresequenz umfasst, die eine Sequenzidentität von mindestens 90% mit dem Polypeptid aufweist, das aus der Aminosäuresequenz, ausgewählt aus den Sequenzen SEQ ID Nr. 1 bis 4, besteht.

11. HIP/PAP-Protein oder eines seiner Derivate für die Verwendung bei Individuen ohne Insulinbedarf zur Vorbeugung oder Behandlung von Atherosklerose und insbesondere koronarer Herzerkrankung und arterieller Erkrankung der unteren Extremitäten,
**dadurch gekennzeichnet, dass** das Derivat eine Aminosäuresequenz umfasst, die eine Sequenzidentität von mindestens 90% mit dem Polypeptid aufweist, das aus der Aminosäuresequenz, ausgewählt aus den Sequenzen SEQ ID Nr. 1 bis 4, besteht.

12. Zusammensetzung, umfassend eine wirksame Menge des HIP/PAP-Proteins oder eines seiner Derivate nach einem der Ansprüche 1 bis 11 im Gemisch mit mindestens einem physiologisch annehmbaren Exzipienten für die Verwendung zur Behandlung oder Vorbeugung von Insulinresistenz bei Individuen ohne Insulinbedarf.

## Claims

1. A HIP/PAP protein, or one of its derivatives thereof, for its use in the treatment or the prevention of insulin resistance in non-insulin-dependent subjects,
**characterized in that** the said derivative comprises an amino acid sequence having a sequence identity of at least 90% with the polypeptide constituted of the amino acid sequence selected among the sequences SEQ ID NO: 1 to 4.

2. The HIP/PAP protein, or one of its derivatives thereof, for its use according to claim 1 **characterized in that** the subjects have a HOMA-B value greater than or equal to 60% and/or a HOMA-IR index of less than 6.

3. The HIP/PAP protein, or one of its derivatives thereof, for its use according to either one of claims 1 or 2, **characterized in that** the subjects have a fasting glycemia of less than 125 mg/dL, and more particularly less than 110 mg/dL fasting.

4. The HIP/PAP protein, or one of its derivatives thereof, for its use according to any one of claims 1 to 3, **characterized in that** the subjects have a glycemia, 2 h after an oral glucose tolerance test, of less than 200 mg/dL, and more particularly less than 140 mg/dL.

5. The HIP/PAP protein, or one of its derivatives thereof, for its use according to any one of the preceding claims, **characterized in that** the subjects have a normal carbohydrate tolerance.

6. The HIP/PAP protein, or one of its derivatives thereof, for its use as claimed in any one of the preceding claims, **characterized in that** the subjects have a normal fasting glycemia.

7. The HIP/PAP protein, or one of its derivatives thereof, for its use according to any one of the preceding claims, **characterized in that** the subjects have at least one of the disorders chosen from the group consisting of: visceral excess weight, obesity, hyperandrogenism, an eating disorder, sarcopenia, hypercatabolism, undernourishment.

8. The HIP/PAP protein, or one of the derivatives thereof, for its use as claimed in any one of the preceding claims, for treating insulin resistance associated with aging.

9. The HIP/PAP protein, or one of its derivatives thereof, for its use in non-insulin-dependent subjects, for increasing muscle development, stimulating increased lean body mass and preventing or limiting muscle catabolism and protein undernourishment,
**characterized in that** the said derivative comprises an amino acid sequence having a sequence identity of at least 90% with the polypeptide constituted of the amino acid sequence selected among the sequences SEQ ID NO: 1 to 4.

10. The HIP/PAP protein, or one of its derivatives thereof, for its use in non-insulin-dependent subjects, for preventing or treating dyslipidemia and in particular hypercholesterolemia,
**characterized in that** the said derivative comprises an amino acid sequence having a sequence identity of at least 90% with the polypeptide constituted of the amino acid sequence selected among the sequences SEQ ID NO: 1 to 4.

11. The HIP/PAP protein, or one of its derivatives thereof, for its use in non-insulin-dependent subjects, for preventing or treating atherosclerosis and in particular coronary artery disease and arteriopathy of the lower limbs,
**characterized in that** the said derivative comprises an amino acid sequence having a sequence identity of at least 90% with the polypeptide constituted of the amino acid sequence selected among the sequences SEQ ID NO: 1 to 4

12. A composition comprising an effective amount of the HIP/PAP protein or of one of its derivatives thereof, as defined in any one of claims 1 to 11, as a mixture with at least one physiologically acceptable excipient, for its use for treating or preventing insulin resistance in non-insulin-dependent subjects.
